(19) Europäisches Patentamt
European Patent Office
Office européen des brevets

(11) Publication number: **0 158 977**
**B1**

(12) **EUROPEAN PATENT SPECIFICATION**

(45) Date of publication of patent specification: **06.09.89**

(21) Application number: **85104391.9**

(22) Date of filing: **11.04.85**

(51) Int. Cl.$^4$: **C 01 B 25/45,** B 01 J 29/04, C 10 G 11/04, C 10 G 49/02, C 07 C 5/02, C 07 C 2/54, C 10 G 35/06, C 07 C 5/22, C 07 C 5/41

(54) **Molecular sieve compositions.**

(30) Priority: **13.04.84 US 599810**

(43) Date of publication of application: **23.10.85 Bulletin 85/43**

(45) Publication of the grant of the patent: **06.09.89 Bulletin 89/36**

(84) Designated Contracting States: **BE DE FR GB IT NL**

(56) References cited: **EP-A-0 043 562**

(73) Proprietor: **UNION CARBIDE CORPORATION**
**39 Old Ridgebury Road**
**Danbury Connecticut 06817 (US)**

(72) Inventor: **Flanigen, Edith Marie**
**502 Woodland Hills Road**
**White Plains New York 10603 (US)**
Inventor: **Lok, Brent Mei Tak**
**17A Deerwood Drive**
**New City New York 10956 (US)**
Inventor: **Marcus, Bonita Kristoffersen**
**49 Meadow Place**
**Rye New York 10580 (US)**
Inventor: **Messina, Celeste Anne**
**20 Somerstown Road**
**Ossining New York 10562 (US)**
Inventor: **Wilson, Stephen Thomas**
**1024 E. Main Street**
**Shrub Oak New York 10588 (US)**

(74) Representative: **Eggert, Hans-Gunther, Dr.**
**Räderscheidtstrasse 1**
**D-5000 Köln 41 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 158 977 B1

## Description

The instant invention relates to a novel class of crystalline microporous molecular sieves, to the method of their preparation and to their use as adsorbents and catalyst. The invention relates to novel molecular sieves which contain framework tetrahedral oxide units of: aluminum; phosphorus; iron and/or titanium; and at least one of cobalt, magnesium, manganese and zinc. These compositions may be prepared hydrothermally from gels containing reactive compounds of the aforementioned elements capable of forming framework tetrahedral oxides, and preferably at least one organic templating agent which functions in part to determine the course of the crystallization mechanism and the structure of the crystalline product.

Molecular sieves of the crystalline aluminosilicate zeolite type are well known in the art and now comprise over 150 species of both naturally occurring and synthetic compositions. In general the crystalline zeolites are formed from corner-sharing $AlO_2$ and $SiO_3$ tetrehedra and are characterized by having pore openings of uniform dimensions, having a significant ion-exchange capacity and being capable of reversibly desorbing an adsorbed phase which is dispersed throughout the internal voids of the crystal without displacing any atoms which make up the permanent crystal structure.

Other crystalline microporous compositions which are not zeolitic, i.e. do not contain $AlO_2$ tetrahedra as essential framework constituents, but which exhibit the ion-exchange and/or adsorption characteristics of the zeolites are also known. Metal organosilicates which are said to possess ion-exchange properties, have uniform pores and are capable of reversibly adsorbing molecules having molecular diameters of about 6Å or less, are reported in U.S. Patent No. 3,941,871 issued March 2, 1976 to Dwyer et al. A pure silica polymorph, silicalite, having molecular sieving properties and a neutral framework containing neither cations nor cation sites is disclosed in U.S. Patent No. 4,061,724 issued December 6, 1977 to R. W. Grose et al.

A recently reported class of microporous compositions and the first framework oxide molecular sieves synthesized without silica, are the crystalline aluminophosphate compositions disclosed in EP—A—0043562 issued January 12, 1982 to Wilson et al. These materials are formed from $AlO_2$ to $PO_2$ tetrahedra and have electrovalently neutral frameworks as in the case of silica polymorphs. Unlike the silica molecular sieve, silicalite, which is hydrophobic due to the absence of extra-structural cations, the aluminophosphate molecular sieves are moderately hydrophilic, apparently due to the difference in electronegativity between aluminum and phosphorus. Their intracrystalline pore volumes and pore diameters are comparable to those known for zeolites and silica molecular sieves.

In EP—A—0103117 there is described a novel class of silicon-substituted aluminophosphates which are both microporous and crystalline. The materials have a three dimensional crystal framework of $PO_2^+$, $AlO_2^-$ and $SiO_2$ tetrahedral units and, exclusive of any alkali metal or calcium which may optionally be present, an as-synthesized empirical chemical composition on an anhydrous basis of:

$$mR : (Si_xAl_yP_z)O_2$$

wherein "R" represents at least one organic templating agent present in the intracrystalline pore system, "m" represents the moles of "R" present per mole of $(Si_xAl_yP_z)O_2$ and has a value of from zero to 0.3, the maximum value in each case depending upon the molecular dimensions of the templating agent and the available void volume of the pore system of the particular silicoaluminophosphate species involved; and "x", "y", and "z" represent the mole fractions of silicon, aluminum and phosphorus, respectively, present as tetrahedral oxides. The minimum value for each of "x", "y", and "z" is 0.01 and preferably 0.02. The maximum value for "x" is 0.98; for "y" is 0.60; and for "z" is 0.52. These silicoaluminophosphates exhibit several physical and chemical properties which are characteristic of aluminosilicate zeolites and aluminophosphates.

In EP—A—0121232 there is described a noval class of titanium-containing molecular sieves (denominated therein as "TAPO" or "TAPOs") whose chemical composition in the as-synthesized and anhydrous form is represented by the unit empirical formula:

$$mR : (Ti_xAl_yP_z)O_2$$

wherein "R" represents at least one organic templating agent present in the intracystalline pore system; "m" represents the moles of "R" present per mole of $(Ti_xAl_yP_z)O_2$ and has a value of between zero and about 5.0; and "x", "y" and "z" represent the mole fractions of titanium, aluminum and phosphorus, respectively, present as tetrahedral oxides.

In EP—A—0132708 there is described a novel class of crystalline metal aluminophosphates (denominated therein as "MeAPO" or "MeAPOs") having three-dimensional microporous framework structures of $MO_2$, $AlO_2$ and $PO_2$ tetrahedral units and having an empirical chemical composition on an anhydrous basis expressed by the formula:

$$mR : (M_xAl_yP_z)O_2$$

wherein "R" represents at least one organic templating agent present in the intracrystalline pore system; "m" represents the moles of "R" present per mole of $(M_xAl_yP_z)O_2$ and has a value of from zero to 0.3; "M" represents at least one metal of the group magnesium, manganese, zinc and cobalt; "x", "y" and "z" represent the mole fraction of the metal "M", aluminum and phosphorus, respectively, present as tetrahedral oxides.

In EP—A—0131946 there is described a noval class of crystalline ferroaluminophosphates (denominated therein as "FAPO" or "FAPOs") having a three-dimensional microporous framework structure of $FeO_2$, $AlO_2$ and $PO_2$ tetrahedral units and having an empirical chemical composition on a anhydrous basis expressed by the formula

$$mR : (Fe_xAl_yP_z)O_2$$

wherein "R" represents at least one organic templating agent present in the intracrystalline pore system, "m" represents the moles of "R" present per mole of $(Fe_xAl_yP_z)O_2$ and has a value of from zero to 0.3; and "x", "y" and "z" represent the mole fraction of the iron, aluminum and phosphorous, respectively, present as tetrahedral oxides.

The instant invention relates to new molecular sieve compositions which comprise framework tetrahedral units of $MO_2^n$, $AlO_2^-$ and $PO_2^+$; where "M" represents iron and/or titanium, and at least one of cobalt, magnesium, manganese and zinc, and where "n" is 0, −1 or −2.

FIG. 1 is a ternary diagram wherein parameters relating to the instant compositions are set forth as mole fractions.

FIG. 2 is a ternary diagram wherein parameters relating to preferred compositions are set forth as mole fractions.

FIG. 3 is a ternary diagram wherein parameters relating to the reaction mixtures employed in the preparation of the compositions of this invention are set forth as mole fractions.

The instant invention relates to a new clas of molecular sieves having a crystal framework structure of $MO_2^n$, $AlO_2^-$ and $PO_2^+$ tetrahedral oxide units wherein "M" represents iron and/or titanium, and at least one of cobalt, magnesium, manganese and zinc, and where "n" is 0 −1 or −2. These new molecular sieves exhibit ion-exchange, adsorption and catalytic properties and, accordingly, find wide use as adsorbents and catalysts. The members of this novel class of compositions have crystal framework structures of $MO_2^n$, $AlO_2^-$ and $PO_2^+$ tetrahedral units and have an empirical chemical composition on an anhydrous basis expressed by the formula:

$$mR : (M_xAl_yP_z)O_2$$

wherein "R" represents at least one organic templating agent present in the intracrystalline pore system; "M" represents at least one element from each of the two classes of: 1) iron and titanium; and 2) cobalt, magnesium, manganese and zinc; "m" represents the molar amount of "R" present per mole of $(M_xAl_yP_z)O_2$ and has a value of zero to about 0.3; and "x", "y" and "z" represent the mole fractions of "M", aluminum and phosphorus, respectively, present as tetrahedral oxides. These molecular sieve compositions comprise crystalline molecular sieves having a three-dimensional microporous framework structure of $MO_2^n$, $AlO_2^-$ and $PO_2^+$ tetrahedral units, wherein "$MO_2^n$" represents tetrahedral units of iron and/or titanium, and at least one of cobalt, magnesium, manganese and zinc.

The molecular sieves of the instant invention will be generally referred to by the acronym, "XAPO" to designate molecular sieves having a three-dimensional microporous framework of $MO_2^n$, $AlO_2^-$ and $PO_2^+$ tetrahedral oxide units as described herein. Actual class members will be identified by denominating the various structural species which make up the XAPO class by assigning a number and by replacing "X" by the elements present as tetrahedral oxides with aluminum and phosphorus and, accordingly, e.g., are identified as "XAPO-i" wherein "i" is an integer. This designation is an arbitrary one and is not intended to denote structural relationship to another material(s) which may also be characterized by a numbering system.

The instant invention relates to a new class of molecular sieves comprising crystal framework structures of $MO_2^n$, $AlO_2^-$ and $PO_2^+$ tetrahedral units where "n' is 0, −1 or −2 and where "M" represents at least one element from each of the two classes of: 1) iron and titanium; and 2) cobalt, magnesium, manganese and zinc. These new molecular sieves exhibit ion-exchange, adsorption and catalytic properties and, accordingly, find wide use as adsorbents and catalysts.

The members of this novel class of compositions have a crystal framework structure of $MO_2^n$, $AlO_2^-$ and $PO_2^+$ tetrahedral oxide units and have an empirical chemical composition on an anhydrous basis expressed by the formula:

$$mR : (M_xAl_yP_z)O_2$$

wherein "R" represents at least one organic templating agent present in the intracrystalline pore system; "M" represents at least two elements from the two classes of: 1) iron and titanium; and 2) cobalt, magnesium, manganese and zinc; "m" represents the molar amount of "R" present per mole of

$(M_xAl_yP_z)O_2$ and has a value of zero to about 0.3; "x", "y" and "z" represent the mole fractions of "M", aluminum and phosphorus, respectively, present as tetrahedral oxides. The mole fractions "x", "y" and "z" are generally defined as being within the pentagonal compositional area defined by points A, B, C, D, and E of the ternary diagram of FIG. 1. Points A, B, C, D, and E of FIG. 1 have the following values for "x", "y" and "z":

| | Mole Fraction | | |
|---|---|---|---|
| Point | x | y | z |
| A | 0.02 | 0.60 | 0.38 |
| B | 0.02 | 0.38 | 0.60 |
| C | 0.39 | 0.01 | 0.60 |
| D | 0.98 | 0.01 | 0.01 |
| E | 0.39 | 0.60 | 0.01 |

In a preferred subclass of the molecular sieves the values of "x", "y" and "z" in the above formula are within the hexagonal compositional area defined by the points a, b, c, d, e, and f of the ternary diagram which is FIG. 2 of the drawings, said points a, b, c, d, e, and f representing the following values for "x", "y" and "z":

| | Mole Fraction | | |
|---|---|---|---|
| Point | x | y | z |
| a | 0.02 | 0.60 | 0.38 |
| b | 0.02 | 0.38 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

The XAPO molecular sieves of this invention are useful as adsorbents, catalysts, ion-exchangers, and the like in much the same fashion as aluminosilicates have been employed heretofore, although their chemical and physical properties are not necessarily similar to those observed for aluminosilicates.

XAPO compositions are generally synthesized by hydrothermal crystallization from a reaction mixture containing reactive sources of "M", aluminum and phosphorus, preferably an organic templating, i.e., structure-directing, agent, preferably a compound of an element of Group VA of the Periodic Table, and/or optionally an alkali metal. The reaction mixture is generally placed in a sealed pressure vessel, preferably lined with an inert plastic material such as polytetrafluoroethylene and heated, preferably under autogenous pressure at a temperature between 50°C and 250°C, and preferably between 100°C and 200°C until crytals of the XAPO product are obtained, usually a period of from hours to several weeks. Typical crystallization times are from about 2 hours to about 30 days with from about 2 hours to about 20 days typically being employed. The product is recovered by any convenient method such as centrifugation or filtration.

In synthesizing the compositions of the instant invention, it is preferred to employ a reaction mixture composition expressed in terms of the molar ratios as follows:

$$aR : (M_xAl_yP_z)O_2 : bH_2O$$

wherein "R" is an organic templating agent; "a" is the amount of organic templating agent "R" and has a value of from zero to about 6 and is preferably an effective amount within the range of greater than zero (0) to about 6; "M" represents at least one element from each of the two classes of: 1) iron and titanium; and 2) cobalt, magnesium, manganese and zinc; "b" has a value of from greater than zero to about 500, preferably between 2 and about 300; and "x", "y" and "z" represent the mole fractions of "M" (iron and/or titanium, and at least one of cobalt, magnesium, manganese and zinc), aluminum and phosphorus, respectively, in the $(M_xAl_yP_z)O_2$ constituent, and each has a value of at least 0.01 with the proviso that "x" has a value of at least 0.02; and "x", "y" and "z" are preferably within the pentagonal compositional area defined by points F, G, H, I and J which is shown in FIG. 3 of the drawings, the points F, G, H, I and J representing the following values for "x", "y" and "z":

| Point | Mole Fraction x | y | z |
|---|---|---|---|
| F | 0.02 | 0.60 | 0.38 |
| G | 0.02 | 0.38 | 0.60 |
| H | 0.39 | 0.01 | 0.60 |
| I | 0.98 | 0.01 | 0.01 |
| J | 0.39 | 0.60 | 0.01 |

In the aforementioned expression of the reaction composition, the reactants are normalized with respect to a total of (x+y+z) = 1.00 mole, whereas in some examples the reaction mixtures may be expressed in terms of molar oxide ratios and may be normalized to 1.00 mole of $P_2O_5$. This latter form is readily converted to the former by routine calculations by dividing the total number of moles of "M", aluminum and phosphorus into the moles of each of "M", aluminum and phosphorus. The moles of template and water may be similarly normalized by dividing by the total moles of "M", aluminum and phosphorus.

In forming the reaction mixture from which the instant molecular sieves are formed the organic templating agent can be any of those heretofore proposed for use in the synthesis of conventional zeolite aluminosilicates. In general these compounds contain elements of Group VA of the Periodic Table of Elements, particularly nitrogen, phosphorus, arsenic and antimony, preferably nitrogen or phosphorus and most preferably nitrogen, which compounds also contain at least one alkyl or aryl group having from 1 to 8 carbon atoms.

Particularly preferred compounds for use as templating agents are the amines, quaternary phosphonium compounds and quaternary ammonium compounds, the latter two being represented generally by the formula $R_4^1X^+$ wherein "X" is phosphorous or nitrogen and each $R^1$ is an alkyl or aryl group containing from 1 to 8 carbon atoms. Polymeric quaternary ammonium salts such as $[(C_{14}H_{32}N_2)(OH)_2]_x$ wherein "x" has a value of at least 2 are also suitably employed. The mono-, di- and tri-amines are advantageously utilized, either alone or in combination with a quaternary ammonium compound or other templating compound. Mixtures of two or more templating agents can either produce mixtures of the desired product or the more strongly directing templating species may control the course of the reaction with the other templating species serving primarily to establish the pH conditions of the reaction gel. Representative templating agents include tetramethylammonium, tetraethylammonium, tetrapropylammonium or tetrabutylammonium ions; tetrapentylammonium ion; di-n-propylamine; tripropylamine; triethylamine; triethanolamine; piperidine; cyclohexylamine; 2-methylpyridine; N,N-dimethylbenzylamine; N,N-dimethylethanolamine; choline; N,N'-dimethylpiperazine; 1,4-diazabicyclo(2,2,2)octane; N-methyldiethanolamine, N-methylethanolamine; N-methylpiperidine; 3-methylpiperidine; N-methylcyclohexylamine; 3-methylpyridine; 4-methylpyridine; quinuclidine; N,N'-dimethyl-1,4-diazabicyclo(2,2,2)octane ion; di-n-butylamine, neopentylamine; di-n-pentylamine; isopropylamine; t-butylamine; ethylenediamine; pyrrolidine; and 2-imidazolidone. Not every templating agent will direct the formation of every species of XAPO, i.e., a single templating agent can, with proper manipulation of the reaction conditions, direct the formation of several XAPO compositions, and a given XAPO composition can be produced using several different templating agents.

The reactive phosphorus source is preferably phosphoric acid, but organic phosphates such as triethyl phosphate have been found satisfactory, and so also have crystalline or amorphous aluminophosphates such as the $AlPO_4$ compositions of EP—A—0043562. Organo-phosphorus compounds, such as tetrabutylphosphonium bromide do not, apparently, serve as reactive sources of phosphorus, but these compounds do function as templating agents. Conventional phosphorus salts such as sodium metaphosphate, may be used, at least in part, as the phosphorus source, but are not preferred.

The preferred aluminum source is either an aluminum alkoxide, such as aluminum isoproproxide, or pseudoboehmite. The crystalline or amorphous aluminophosphates which are a suitable source of phosphorus are, of course, also suitable sources of aluminum. Other sources of aluminum used in zeolite synthesis, such as gibbsite, sodium aluminate and aluminum trichloride, can be employed but are not preferred.

The reactive source of iron can be introduced into the reaction system in any form which permits the formation *in situ* of reactive ferrous or ferric ions capable of forming the framework tetrahedral unit of $FeO_2$. Advantageously, iron compounds including oxides, hydroxides, sulfates, acetates, nitrates and the like may be employed. Other reactive sources of iron include freshly precipitated iron oxide and gamma-FeOOH may be employed.

The reactive source of titanium can be introduced into the reaction system in any form which permits the formation *in situ* of reactive titanium capable of forming the $TiO_2$ framework tetrahedral unit. Titanium compounds which may be employed herein include titanium alkoxides, titanium halides, e.g., titanium tetrachloride, water-soluble titanates, titanium chelates and the like.

The reactive source of cobalt, magnesium, manganese and zinc can be introduced into the reaction system in any form which permits the formation *in situ* of reactive ions of cobalt, magnesium, manganese

and zinc capable of forming the framework tetrahedral units of such. Advantageously, salts, oxides, alkoxides or hydroxides of metals are employed such as cobalt chloride hexahydrate, alpha cobaltous iodide, cobaltous sulfate, cobalt acetate, cobaltous bromide, cobaltous chloride, zinc acetate, zinc bromide, zinc formate, zinc iodide, zinc sulfate heptahydrate, magnesium acetate, magnesium bromide, magnesium chloride, magnesium iodide, magnesium nitrate, magnesium sulfate, manganous acetate, manganous bromide, manganous sulfate and the like.

While not essential to the synthesis of XAPO compositions, stirring or other moderate agitation of the reaction mixture and/or seeding the reaction mixture with seed crystals of either the XAPO species to be produced or a topologically similar aluminophosphate, aluminosilicate or molecular sieve composition, facilitates the crystallization procedure.

After crystallization the XAPO product may be isolated and advantageously washed with water and dried in air. The as-synthesized XAPO generally contains within its internal pore system at least one form of the templating agent employed in its formation. Most commonly the organic moiety is present, at least in part, as a charge-balancing cation as is generally the case with as-synthesized aluminosilicate zeolites prepared from organic-containing reaction systems. It is possible, however, that some or all of the organic moiety is an occluded molecular species in a particular XAPO species. As a general rule the templating agent, and hence any occluded organic species, is too large to move freely through the pore system of the XAPO product and must be removed by calcining the XAPO at temperatures of 200°C to 700°C to thermally degrade the organic species. In a few instances the pores of the XAPO product are sufficiently large to permit transport of the templating agent, particularly if the latter is a small molecule, and accordingly complete or partial removal thereof can be accomplished by conventional desorption procedures such as carried out in the case of zeolites. It will be understood that the term "as-synthesized" as used herein does not include the condition of the XAPO phase wherein the organic moiety occupying the intracrystalline pore system as a result of the hydrothermal crystallization process has been reduced by post-synthesis treatment such that the value of "m" in the composition formula

$$mR : (M_xAl_yP_z)O_2$$

has a value of less than 0.02. The other symbols of the formula are as defined hereinabove. In those preparations in which an alkoxide is employed as the source of "M", aluminum or phosphorus, the corresponding alcohol is necessarily present in the reaction mixture since it is a hydrolysis product of the alkoxide. It has not been determined whether this alcohol participates in the synthesis process as a templating agent. For the purposes of this application, however, this alcohol is arbitrarily omitted from the class of templating agents, even if it is present in the as-synthesized XAPO material.

Since the present XAPO compositions are formed from $MO_2^n$, $AlO_2$ and $PO_2$ tetrahedral units which, respectively, have a net charge of "n" (0, −1 or −2), −1 and +1, the matter of cation exchangeability is considerably more complicated than in the case of zeolitic molecular sieves in which, ideally, there is a stoichiometric relationship between $AlO_2^-$ tetrahedra and charge-balancing cations. In the instant compositions, an $AlO_2^-$ tetrahedron can be balanced electrically either by association with a $PO_2^+$ tetrahedron or a simple cation such as an alkali metal cation, a proton ($H^+$), a cation of "M" present in the reaction mixture, or an organic cation derived from the templating agent. Similarly a $MO_2^n$ tetrahedron can be balanced electrically by association with $PO_2^+$ tetrahedra, a cation of "M" present in the reaction mixture, a simple cation such as an alkali metal cation, a proton ($H^+$), organic cations derived from the templating agent, or other divalent or polyvalent metal cations introduced from an extraneous source. It has also been postulated that non-adjacent $AlO_2^-$ and $PO_2^+$ tetrahedral pairs can be balanced by $Na^+$ and $OH^-$, respectively [Flanigen and Grose, Molecular Sieve Zeolites-I, ACS, Washington, DC (1971)].

The XAPO compositions of the present invention may exhibit cation-exchange capacity when analyzed using ion-exchange techniques heretofore employed with zeolite aluminosilicates and have pore diameters which are inherent in the lattice structure of each species and which are at least about 3Å in diameter. Ion exchange of XAPO compositions is ordinarily possible only after any organic moiety derived from the template, present as a result of synthesis has been removed from the pore system. Dehydration to remove water present in the as-synthesized XAPO compositions can usually be accomplished, to some degree at least, in the usual manner without removal of the organic moiety, but the absence of the organic species greatly facilitates adsorption and desorption procedures. The XAPO materials have various degrees of hydrothermal and thermal stability, some being quite remarkable in this regard, and function well as molecular sieve adsorbents and hydrocarbon conversion catalysts or catalyst bases.

In preparing the XAPO composition it is preferred to use a stainless steel reaction vessel lined with an inert plastic material, e.g., polytetrafluoroethylene, to avoid contamination of the reaction mixture. In general, the final reaction mixture from which each XAPO composition is crystallized is prepared by forming mixtures of less than all of the reagents and therafter incorporating into these mixtures additional reagents either singly or in the form of other intermediate mixtures of two or more reagents. In some instances the reagents admixed retain their identity in the intermediate mixture and in other cases some or all of the reagents are involved in chemical reactions to produce new reagents. The term "mixture" is applied in both cases. Further, it is preferred that the intermediate mixtures as well as the final reaction mixtures be stirred until substantially homogeneous.

X-ray patterns of reaction products are obtained by X-ray analysis using standard X-ray powder diffraction techniques. The radiation source is a high-intensity, copper target, X-ray tube operated at 50 KV and 40 mA. The diffraction pattern from the copper K-alpha radiation and graphite monochromator is suitably recorded by an X-ray spectrometer scintillation counter, pulse height analyzer and strip chart recorder. Flat compressed powder samples are scanned at 2° (2 theta) per minute, using a two second time constant. Interplanar spacings (d) in Angstrom units are obtained from the position of the diffraction peaks expressed as 2θ where θ is the Bragg angle as observed on the strip chart. Intensities are determined from the heights of diffraction peaks after subtracting background, "$I_o$" being the intensity of the strongest line or peak, and "I" being the intensity of each of the other peaks. Alternatively, the X-ray patterns may be obtained by use of computer based techniques using Copper K-alpha radiation with Sieman's Type K-805 X-ray sources and with Siemens D-500 X-ray powder diffractometers available form Siemens Corporation, Cherry Hill, NJ.

As will be understood by those skilled in the art the determination of the parameter 2 theta is subject to both human and mechanical error, which in combination, can impose an uncertainty of about ±0.4° on each reported value of 2 theta. This uncertainty is, of course, also manifested in the reported values of the d-spacings, which are calculated from the 2 theta values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m, w and vw which represent very strong, strong, medium, weak and very weak, respectively.

In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

The molecular sieves of the instant invention may be characterized by their x-ray powder diffraction patterns and such may have one of the x-ray patterns set forth in the following Tables A through V, wherein said x-ray patterns are for both the as-synthesized and calcined forms unless otherwise noted:

TABLE A (XAPO-5)

| 2θ | d(Å) | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

TABLE B (XAPO-11)

| 2θ | d(Å) | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

TABLE C (XAPO-14)

| 2θ | d(Å) | Relative Intensity |
|---|---|---|
| 8.6 - 8.9 | 10.3 - 9.93 | vs |
| 13.0 | 6.81 | w |
| 21.9 - 22.2 | 4.06 - 4.00 | w |
| 25.4 | 3.51 | w |
| 27.5 | 3.24 | w |
| 29.7 | 3.01 | w |

TABLE D (XAPO-16)

| 2θ | d(Å) | Relative Intensity |
|---|---|---|
| 11.3 - 11.6 | 7.83 - 7.63 | m - vs |
| 18.7 - 18.9 | 4.75 - 4.70 | w - s |
| 21.9 - 22.3 | 4.06 - 3.99 | m - vs |
| 26.5 - 27.0 | 3.363 - 3.302 | w - m |
| 29.7 - 30.05 | 3.008 - 2.974 | w - m |

TABLE E (XAPO-17)

| 2θ | d(Å) | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

TABLE F (XAPO-18)

| 2θ | d(Å) | Relative Intensity |
|---|---|---|
| 9.6 - 9.65 | 9.21 - 9.16 | vs |
| 15.5 - 15.55 | 5.72 - 5.70 | m |
| 16.9 - 17.1 | 5.25 - 5.19 | m |
| 20.15 - 20.25 | 4.41 - 4.39 | m |
| 20.95 - 21.05 | 4.24 - 4.22 | m |
| 31.8 - 32.5 | 2.814 - 2.755 | m |

TABLE G (XAPO-20)

| 2θ | d(Å) | Relative Intensity |
|---|---|---|
| 13.7 - 14.25 | 6.46 - 6.22 | m - vs |
| 19.55 - 20.0 | 4.54 - 4.44 | w - s |
| 24.05 - 24.5 | 3.70 - 3.63 | m - vs |
| 34.3 - 35.0 | 2.614 - 2.564 | vw - w |
| 42.5 - 43.0 | 2.127 - 2.103 | vw - w |

TABLE H (XAPO-31)

| 2θ | d(Å) | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

TABLE J* (XAPO-33)

| 2θ | d(Å) | Relative Intensity |
|---|---|---|
| 9.25 - 9.55 | 9.56 - 9.26 | w - m |
| 12.5 - 12.9 | 7.08 - 6.86 | vs |
| 16.9 - 17.3 | 5.25 - 5.13 | w - m |
| 20.45 - 20.9 | 4.34 - 4.25 | w - m |
| 23.85 - 24.25 | 3.73 - 3.67 | w - m |
| 26.05 - 26.35 | 3.42 - 3.38 | w - m |
| 27.3 - 27.6 | 3.27 - 3.23 | vs |

* as-synthesized form

TABLE K* (XAPO-33)

| 2θ | d(Å) | Relative Intensity |
|---|---|---|
| 13.15 - 13.4 | 6.73 - 6.61 | vs |
| 18.05 - 18.35 | 4.91 - 4.83 | m |
| 18.4 - 18.6 | 4.82 - 4.77 | m |
| 26.55 - 26.7 | 3.36 - 3.34 | m |
| 32.0 - 32.1 | 2.80 - 2.79 | m |

* calcined form

TABLE L (XAPO-34)

| 2θ | d(Å) | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

TABLE M (XAPO-35)

| 2θ | d(Å) | Relative Intensity |
|---|---|---|
| 10.8 - 11.1 | 8.19 - 7.97 | m |
| 17.2 - 17.4 | 5.16 - 5.10 | s - vs |
| 21.0 - 21.25 | 4.23 - 4.18 | m - s |
| 21.8 - 22.0 | 4.08 - 4.04 | vs |
| 31.8 - 32.2 | 2.814 - 2.788 | m |

TABLE N (XAPO-36)

| 2θ | d(Å) | Relative Intensity |
|---|---|---|
| 7.7 - 7.9 | 11.5 - 11.2 | vs |
| 16.2 - 16.6 | 5.47 - 5.34 | w - m |
| 18.9 - 19. 3 | 4.70 - 4.60 | m - s |
| 20.6 - 20.8 | 4.31 - 4.27 | w - s |
| 21.8 - 22.0 | 4.08 - 4.04 | m |
| 22.2 - 22.5 | 4.00 - 3.95 | w - m |

TABLE O (XAPO-37)

| 2θ | d(Å) | Relative Intensity |
|---|---|---|
| 6.1 - 6.3 | 14.49 - 14.03 | vs |
| 15.5 - 15.7 | 5.72 - 5.64 | w - m |
| 18.5 - 18.8 | 4.80 - 4.72 | w - m |
| 23.5 - 23.7 | 3.79 - 3.75 | w - m |
| 26.9 - 27.1 | 3.31 - 3.29 | w - m |

TABLE P (XAPO-39)

| 2θ | d(Å) | Relative Intensity |
|---|---|---|
| 9.4 - 9.6 | 9.41 - 9.21 | w - m |
| 13.3 - 13.6 | 6.66 - 6.51 | m - vs |
| 18.0 - 18.4 | 4.93 - 4.82 | m |
| 21.2 - 21.5 | 4.19 - 4.13 | m - s |
| 22.5 - 23.0 | 3.95 - 3.87 | s - vs |
| 30.2 - 30.5 | 2.96 - 2.93 | w - m |

TABLE Q (XAPO-40)

| 2θ | d(Å) | Relative Intensity |
|---|---|---|
| 7.5 - 7.7 | 11.79 - 11.48 | vw - m |
| 8.0 - 8.1 | 11.05 - 10.94 | s - vs |
| 12.4 - 12.5 | 7.14 - 7.08 | w - vs |
| 13.6 - 13.8 | 6.51 - 6.42 | m - s |
| 14.0 - 14.1 | 6.33 - 6.28 | w - m |
| 27.8 - 28.0 | 3.209 - 3.187 | w - m |

TABLE R (XAPO-41)

| 2θ | d(Å) | Relative Intensity |
|---|---|---|
| 13.6 - 13.8 | 6.51 - 6.42 | w - m |
| 20.5 - 20.6 | 4.33 - 4.31 | w - m |
| 21.1 - 21.3 | 4.21 - 4.17 | vs |
| 22.1 - 22.3 | 4.02 - 3.99 | m - s |
| 22.8 - 23.0 | 3.90 - 3.86 | m |
| 23.1 - 23.4 | 3.82 - 3.80 | w - m |
| 25.5 - 25.9 | 3.493 - 3.440 | w - m |

TABLE S (XAPO-42)

| 2θ | d(Å) | Relative Intensity |
|---|---|---|
| 7.15 - 7.4 | 12.36 - 11.95 | m - vs |
| 12.5 - 12.7 | 7.08 - 6.97 | m - s |
| 21.75 - 21.9 | 4.09 - 4.06 | m - s |
| 24.1 - 24.25 | 3.69 - 3.67 | vs |
| 27.25 - 27.4 | 3.273 - 3.255 | s |
| 30.05 - 30.25 | 2.974 - 2.955 | m - s |

TABLE T (XAPO-44)

| 2θ | d(Å) | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

TABLE U (XAPO-46)

| 2θ | d(Å) | Relative Intensity |
|---|---|---|
| 7.2 - 8.1 | 12.3 - 10.9 | vs |
| 21.2 - 21.8 | 4.19 - 4.08 | w - m |
| 22.5 - 23.0 | 3.95 - 3.87 | vw - m |
| 26.6 - 27.2 | 3.351 - 3.278 | vw - w |
| 28.5 - 29.0 | 3.132 - 3.079 | vw - w |

TABLE V (XAPO-47)

| 2θ | d(Å) | Relative Intensity |
|---|---|---|
| 9.4 | 9.41 | vs |
| 15.9 - 16.0 | 5.57 - 5.54 | w - m |
| 20.5 - 20.6 | 4.33 - 4.31 | s |
| 24.5 - 24.7 | 3.63 - 3.60 | w |
| 25.8 - 25.9 | 3.45 - 3.44 | w |
| 30.4 - 30.5 | 2.940 - 2.931 | w |

The following examples are provided to further illustrate the invention and are not provided to be limiting thereof:

Example 1

(Preparation of FeMgAPO-5)

a) FeMgAPO-5 is prepared from a reaction mixture having a composition, expressed in terms of the molar oxide ratios of the components of the reaction mixture, of: 1.0—2.0 TPA : 0.05—0.2 $Fe_2O_q$ : 0.1—0.4 MgO : 0.5—1.0 $Al_2O_3$ : 0.5—1.0 $P_2O_5$ : 40—100 $H_2O$ where "TPA" denotes tripropylamine and "q" denotes the oxidation state of iron.

The reaction mixture is digested by placing the reaction mixture in a sealed stainless steel pressure vessel and heating it at an effective temperature and for an effective time to produce the FeMgAPO-5 product. Solids are recovered by filtration, washed with water and dried in air at room temperature.

The FeMgAPO-5 product's chemical analysis shows the FeMgAPO-5 product contains iron, magnesium, aluminum and phosphorus in amounts within the pentagonal compositional area defined by points A, B, C, D, and E of FIG. 1.

The x-ray powder diffraction pattern of an FeMgAPO-5 product is characterized by the following data:

| 2θ | d(Å) | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

b) The x-ray powder diffraction pattern for a calcined FeMgAPO-5 is also characterized by the X-ray pattern of part a).

c) When the calcined FeMgAPO-5 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 7 |
| $O_2$ | 3.46 | 750 | -183 | 10 |
| Neopentane | 6.2 | 700 | 24 | 4 |
| $H_2O$ | 2.65 | 4.3 | 24 | 4 |
| $H_2O$ | 2.65 | 20.0 | 24 | 12 |

*typical amount adsorbed

The pore diameter of FeMgAPO-5 is greater than 6.2Å.

Example 2

(Preparation of FeMnAPO-11)

a) FeMnAPO-11 is prepared from a reaction mixture having a composition, expressed in terms of the molar oxide ratios of the components of the reaction mixture, of: 1.0—2.0 DPA : 0.05—0.2 $Fe_2O_q$ : 0.1—0.4 MnO : 0.5—1.0 $Al_2O_3$ : 0.5—1.0 $P_2O_5$ : 40—100 $H_2O$ where "DPA" denotes di-n-propylamine and "q" denotes the oxidation state(s) of iron.

The reaction mixture is digested by placing the reaction mixture in a sealed stainless steel pressure vessel and heating it at an effective temperature and for an effective time to produce FeMnAPO-11 product. Solids are then recovered by filtration, washed with water and dried in air at room temperature.

The FeMnAPO-11 product's chemical analysis shows the FeMnAPO-11 product contains iron, manganese, aluminum and phosphorus in amounts within the pentagonal compositional area defined by points A, B, C, D, and E of FIG. 1.

The x-ray powder diffraction pattern of an FeMnAPO-11 product is characterized by the following data:

| 2θ | d(Å) | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

b) The x-ray powder diffraction pattern for a calcined FeMnAPO-11 is also characterized by the X-ray pattern of part a).

c) When the calcined FeMnAPO-11 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 5 |
| $O_2$ | 3.46 | 750 | -183 | 6 |
| Cyclohexane | 6.0 | 90 | 24 | 4 |
| $H_2O$ | 2.65 | 4.3 | 24 | 6 |
| $H_2O$ | 2.65 | 20 | 24 | 8 |

*typical amount adsorbed

The pore diameter of FeMnAPO-11 is about 6Å.

Example 3

(Preparation of FeCoMgAPO-17)

a) FeCoMgAPO-17 is prepared from a reaction mixture having a composition, expressed in terms of the molar oxide ratios of the components of the reaction mixture, of: 1.0—2.0 QN : 0.05—0.2 $M_2O_q$ : 0.5—1.0 $Al_2O_3$ : 0.5—1.0 $P_2O_5$ : 40—100 $H_2O$ where "QN" denotes quinuclidine and "q" denotes the oxidation states of "M" (iron, cobalt, and magnesium).

The reaction mixture is digested by placing the reaction mixture in a sealed stainless steel pressure vessel and heating it at an effective temperature and for an effective time to produce FeCoMgAPO-17 product. Solids are then recovered by filtration, washed with water and dried in air at room temperature. The FeCoMgAPO-17 product's chemical analysis shows the FeCoMgAPO-17 product contains iron, cobalt, magnesium, aluminum and phosphorus in amounts within the pentagonal compositional area defined by points A, B, C, D, and E of FIG. 1.

The x-ray powder diffraction pattern of a FeCoMgAPO-17 product is characterized by the following data:

| 2θ | d(Å) | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

b) The x-ray powder diffraction pattern for a calcined FeCoMgAPO-17 is also characterized by the X-ray pattern of part a).

c) When the calcined FeCoMgAPO-17 of part (b) is utilized in adsorption capacity studies using a standard McBain-Bakr gravimetric adsorption apparatus the measurements are made on a sample after activation of 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 10 |
| $O_2$ | 3.46 | 750 | -183 | 12 |
| n-butane | 4.3 | 100 | 24 | 4 |
| $H_2O$ | 2.65 | 4.3 | 24 | 13 |
| $H_2O$ | 2.65 | 20 | 24 | 14 |

*typical amount adsorbed

The pore diameter of FeCoMgAPO-17 is about 4.3Å.

Example 4

(Preparation of TiZnAPO-31)

a) TiZnAPO-31 is prepared from a reaction mixture having a composition, expressed in terms of the molar oxide ratios of the components of the reaction mixture, of: 1.0—2.0 DPA : 0.05—0.2 $M_2O_q$ : 0.5—1.0 $Al_2O_3$ : 0.5—1.0 $P_2O_5$ : 40—100 $H_2O$ wherein "DPA" denotes di-n-propylamine and "q" denotes the oxidation state(s) of "M" (titanium and zinc).

The reaction mixture is seeded with crystals of $AlPO_4$-31 (U.S. Patent No. 4,310,440) and digested by placing the reaction mixture in a sealed stainless steel pressure vessel and heating it at an effective temperature and for an effective time to produce TiZnAPO-31 product. Solids are then recovered by filtration, washed with water and dried in air at room temperature.

The TiZnAPO-31 product's chemical analysis shows the TiZnAPO-31 product contains titanium, zinc, aluminum and phosphorus in amounts within the pentagonal compositional area defined by points A, B, C, D, and E of FIG. 1.

The x-ray powder diffraction pattern of a TiZnAPO-31 product is characterized by the following data:

data:

| 2θ | d(Å) | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

b) The x-ray powder diffraction pattern for a calcined TiZnAPO-31 is also characterized by the X-ray pattern of part a).

c) When the calcined TiZnAPO-31 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 4 |
| $O_2$ | 3.46 | 750 | -183 | 6 |
| Cyclohexane | 6.0 | 90 | 24 | 3 |
| Neopentane | 6.2 | 700 | 24 | 3 |
| $H_2O$ | 2.65 | 4.3 | 24 | 3 |
| $H_2O$ | 2.65 | 20 | 24 | 10 |

* typical amount adsorbed

The pore diameter of TiZnAPO-31 is greater than about 6.2 Å.

Example 5

(Preparation of FeTiCoAPO-34)

a) FeTiCoAPO-34 is prepared from a reaction mixture having a composition, expressed in terms of the molar oxide ratios of the components of the reaction mixture, of: 1.0—2.0 TEAOH : 0.05—0.2 $M_2O_q$ : 0.5—1.0 $Al_2O_3$ : 0.5—1.0 $P_2O_5$ : 40—100 $H_2O$ where "TEAOH" denotes tetraethylammonium hydroxide and "q" denotes the oxidation state(s) of "M" (iron titanium and cobalt).

The reaction mixture is digested by placing the reaction mixture in a sealed stainless steel pressure vessel and heating it at an effective temperature and for an effective time to produce FeTiCoAPO-34 product. The solids are recovered by filtration, washed with water and dried in air at room temperature.

The FeTiCoAPO-34 product's chemical analysis shows the FeTiCoAPO-34 product contains iron, titanium, cobalt, aluminum and phosphorus in amounts within the pentagonal compositional area defined by points A, B, C, D, and E of FIG. 1.

The x-ray powder diffraction pattern of an FeTiCoAPO-34 product is characterized by the following data:

| 2θ | d(Å) | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

b) The x-ray powder diffraction pattern for a calcined FeTiCoAPO-34 is also characterized by the X-ray pattern of part a).

c) When the calcined FeTiCoAPO-34 of part (b) is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 13 |
| $O_2$ | 3.46 | 750 | -183 | 18 |
| n-hexane | 4.3 | 100 | 24 | 6 |
| $H_2O$ | 2.65 | 4.3 | 24 | 15 |
| $H_2O$ | 2.65 | 20 | 24 | 21 |

*typical amount adsorbed

The pore diameter of FeTiCoAPO-34 is about 4.3 Å.

Example 6

(Preparation of FeMnAPO-44)

a) FeMnAPO-44 is prepared from a reaction mixture having a composition, expressed in terms of the molar oxide ratios of the components of the reaction mixture of: 1.0—2.0 CHA : 0.05—0.2 $M_2O_q$ : 0.5—1.0 $Al_2O_3$ : 0.5—1.0 $P_2O_5$ : 40—100 $H_2O$ where "CHA" denotes cyclohexylamine and "q" denotes the oxidation state of "M" (iron and manganese).

The reaction mixture is digested by placing the reaction mixture in a sealed stainless steel pressure vessel and heating it at an effective temperature and for an effective time to produce the FeMnAPO-44 product. Solids are then recovered by filtration, washed with water and dried in air at room temperature.

The FeMnAPO-44 product's chemical analysis shows the FeMnAPO-44 product contains iron, manganese, aluminum and phosphorus in amounts within the pentagonal compositional area defined by points A, B, C, D, and E of FIG. 1.

The x-ray powder diffraction pattern of an FeMnAPO-44 product is characterized by the following data:

| 2θ | d(Å) | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

b) When the calcined FeMnAPO-44 is utilized in adsorption capacity studies using a standard McBain-Bakr gravimetric adsorption apparatus the measurements are made on a sample after activation at 350°C in vacuum. The following data are used in the adsorption studies:

| Adsorbate | Kinetic Diameter (Å) | Pressure (Torr) (× 1.33 mbar) | Temp, °C | Wt. % Adsorbed* |
|---|---|---|---|---|
| $O_2$ | 3.46 | 100 | -183 | 13 |
| $O_2$ | 3.46 | 750 | -183 | 16 |
| n-hexane | 4.3 | 100 | 24 | 2 |
| $H_2O$ | 2.65 | 4.3 | 24 | 1.5 |
| $H_2O$ | 2.65 | 20 | 24 | 17 |

*typical amount adsorbed

The pore diameter of FeMnAPO-44 is about 4.3Å.

Process Applications

The XAPO compositions of the present invention are, in general, hydrophilic and adsorb water preferentially over common hydrocarbon molecules such as paraffins, olefins and aromatic species, e.g., benzene, xylenes and cumene. Thus the present molecular sieve compositions as a class are useful as desiccants in such adsorption separation/purification processes as natural gas drying, cracked gas drying. Water is also preferentially adsorbed over the so-called permanent gases such as carbon dioxide, nitrogen, oxygen and hydrogen. These XAPOs are therefore suitably employed in the drying of reformer hydrogen streams and in the drying of oxygen, nitrogen or air prior to liquifaction.

The present XAPO compositions also exhibit novel surface selectivity characteristics which render them useful as catalyst or catalyst bases in a number of hydrocarbon conversion and oxidative combustion reactions. They can be impregnated or otherwise loaded with catalytically active metals by methods well known in the art and used, for example, in fabricating catalyst compositions having silica or alumina bases. Of the general class, those species having pores larger than about 4Å are preferred for catalytic applicatons.

Among the hydrocarbon conversion reactions catalyzed by XAPO compositions are cracking, hydrocracking, alkylation for both the aromatic and isoparaffin types, isomerization including xylene isomerization, polymerization, reforming, hydrogenation, dehydrogenation, transalkylation, dealkylation, hydrodecylization and dehydrocyclization.

Using XAPO catalyst compositions which contain a hydrogenation promoter such as platinum or palladium, heavy petroleum residual stocks, cyclic stocks and other hydrocrackable charage stocks, can be hydrocracked at temperatures in the range of 204.4°C (400°F) to 440.6°C (825°F) using molar ratios of hydrogen to hydrocarbon in the range between 2 and 80, pressures between 1.702 bar and 242.16 bar (10 and 3500 p.s.i.g.), and a liquid hourly space velocity (LHSV) of from 0.1 to 20, preferably 1.0 to 10.

The XAPO catalyst compositions employed in hydrocracking are also suitable for use in reforming processes in which the hydrocarbon feedstocks contact the catalyst at temperatures of from about 371.1°C (700°F) to 537.8°C (1000°F) hydrogen pressures of from 7.9 to 35.4 bar (100 to 500 p.s.i.g.), LHSV values in the range of 0.1 to 10 and hydrogen to hydrocarbon molar ratios in the range of 1 to 20, preferably between 4 and 12.

These same catalysts, i.e. those containing hydrogenation promoters, are also useful in hydroisomerizations processes in which feedstocks such a normal paraffins are converted to saturated branched chain isomers. Hydroisomerization is carried out at a temperature of from about 93.3°C (200°F) to 315.5°C (600°F) preferably 148.9°C (300°F) to 287.8°C (550°F) with an LHSV value of from about 0.2 to 1.0. Hydrogn (H) is supplied to the reactor in admixture with the hdrocarbon (Hc) feedstock in molar properties (H/Hc) of between 1 and 5.

At somewhat higher temperatures, i.e. from about 343.3°C (650°F) to 337.8°C (1000°F), preferably 454.4°C (850°F) to 510.0°C (950°F) and usually at somewhat lower pressures within the range of about 2.04 to 4.46 (15 to 50 p.s.i.g.), the same catalyst compositions are used to hydroisomerize normal paraffins. Preferably the paraffin feedstock comprises normal paraffins having a carbon number range of $C_7$—$C_{20}$. Contact time between the feedstock and the catalyst is generally relatively short to avoid undesireable side reactions such as olefin polymerization and paraffin cracking. LHSV values in the range of 0.1 to 10, preferably 1.0 to 6.0 are suitable.

The unique crystal structure of the present XAPO catalysts and their availability in a form totally void of alkali metal content favor their use in the conversion of alkylaromatic compounds, particularly the catalytic disproportionation of toluene, ethylene, trimethyl benzenes, tetramethyl benzenes and the like. In the disproportionation process, isomerization and transalkylation can also occur. Group VIII noble metal adjuvants alone or in conjunction with Group VI-B metals such as tungsten, molybdenum and chromium are preferably included in the catalyst composition in amounts of from about 3 to 15 weight-% of the overall composition.

Extraneous hydrogen can, but need not, be present in the reaction zone which is maintained at a temperature of from about 204.4 to 398.9°C (400 to 750°F), pressures in the range of 7.9 to 138.8 bar (100 to 2000 p.s.i.g.) and LHSV values in the range of 0.1 to 15.

Catalytic cracking processes are preferably carried out with XAPO compositions using feedstocks such as gas oils, heavy naphthas, deasphalted crude oil residua, etc., with gasoline being the principal desired product. Temperature conditions of 454.4 to 593.3°C (850 to 1100°F) LHSV values of 0.5 to 10 and pressure conditions of from about 1.013 to 4.46 bar (0 to 50 p.s.i.g.) are suitable.

Dehydrocyclization reactions employing paraffinic hydrocarbon feedstocks, preferably normal paraffins having more than 6 carbon atoms, to form benzene, xylenes, toluene and the like are carried out using essentially the same reaction conditions as for catalytic cracking. For these reactions it is preferred to use the XAPO catalyst in conjunction with a Group VIII non-noble metal cation such as cobalt and nickel.

In catalytic dealkylation wherein it is desired to cleave paraffinic side chains from aromatic nuclei without substantially hydrogenating the ring structure, relatively high temperatures in the range of about 426.7 to 537.8°C (800°—1000°F) are employed at moderate hydrogen pressures of about 21.68 to 69.91 bar (300—1000 p.s.i.g.), other conditions being similar to those described above for catalytic hydrocracking. Preferred catalysts are of the same type described above in connection with catalytic dehydrocyclization. Particularly desirable dealkylation reactions contemplated herein include the conversion of methylnaphthalene to naphthalene and toluene and/or xylenes to benzene.

In catalytic hydrofining, the primary objective is to promote the selective hydrodecomposition of organic sulfur and/or nitrogen compounds in the feed, without substantially affecting hydrocarbon molecules therein. For this purpose it is preferred to employ the same general conditions described above for catalytic hydrocracking, and catalysts of the same general nature described in connection with dehydrocyclization operations. Feedstocks include gasoline fractions, kerosenes, jet fuel fractions, diesel fractions, light and heavy gas oils, deasphalted crude oil residua and the like any of which may contain up to about 5 weight-percent of sulfur and up to about 3 weight-percent of nitrogen.

Similar conditions can be employed to effect hydrofining, i.e. denitrogenation and desulfurization, of hydrocarbon feeds containing substantial proportions of organonitrogen and organosulfur compounds. It is generally recognized that the presence of substantial amounts of such constituents markedly inhibits the activity of hydrocracking catalysts. Consequently, it is necessary to operate at more extreme conditions when it is desired to obtain the same degree of hydrocracking conversion per pass on a relatively nitrogenous feed than are required with a feed containing less organonitrogen compounds. Consequently, the conditions under which denitrogenation, desulfurization and/or hydrocracking can be most expeditiously accomplished in any given situation are necessarily determined in view of the characteristics of the feedstocks in particular the concentration of organonitrogen compounds in the feedstock. As a result of the effect of organonitrogen compounds on the hydrocracking activity of these compositions it is not at all unlikely that the conditions most suitable for denitrogenation of a given feedstock having a relatively high organonitrogen content with minimal hydrocracking, e.g., less than 20 volume percent of fresh feed per pass, might be the same as those preferred for hydrocracking another feedstock having a lower concentration of hydrocracking inhibiting constituents e.g., organonitrogen compounds. Consequently, it has become the practice in this art to establish the conditions under which a certain fed is to be contacted on the basis of preliminary screening tests with the specific catalyst and feedstock.

Isomerization reactions are carried out under conditions similar to those described above for reforming, using somewhat more acidic catalysts. Olefins are preferably isomerized at temperatures of 260.0 to 482.2°C (500°—900°F), while paraffins naphthenes and alkyl aromatics are isomerized at temperatures of 371.1 to 537.8°C (700°—1000°F). Particularly desirable isomerization reactions contemplated herein include the conversion of n-heptene and/or n-octane to isoheptanes, iso-octanes, butane to iso-butane, methylcyclopentane to cyclohexane, meta-xylene and/or ortho-xylene to paraxylene, 1-butene to 2-butene and/or isobutene, n-hexene to isohexene, cyclohexene to methylcyclopentene etc. The preferred form of the catalyst is a combination of the XAPO with polyvalent metal compounds (such as sulfides) of metals of Group II-A, Group II-B and rare earth metals. For alkylation and dealkylation processes the XAPO compositions having pores of at least 5Å are preferred. When employed for dealkylation of alkyl aromatics, the temperature is usually at least 176.7°C (350°F) and ranges up to a temperature at which substantial cracking of the feedstock or conversion products occurs, generally up to about 371.1°C (700°F). The temperature is preferably at least 232.2°C (450°F) and not greater than the critical temperature of the compound undergoing dealkylation. Pressure conditions are applied to retain at least the aromatic feed in the liquid state. For alkylation the temperature can be as low as 121.1°C (250°F) but is preferably at least 176.7°C (350°F). In the alkylation of benzene, toluene and xylene, the preferred alkylating agents are olefins such as ethylene and propylene.

**Claims**

1. Crystalline molecular sieves having three-dimensional microporous framework structures of $MO_2^n$, $AlO_2$, and $PO_2$ tetrahedral oxide units having an empirical chemical composition on an anhydrous basis expressed by the formula:

$$mR: (M_xAl_yP_z)O_2$$

wherein "R" represents at least one organic templating agent present in the intracrystalline pore system; "M" represents at least one element from each of the classes of: 1) iron and titanium; and 2) cobalt, magnesium, manganese and zinc; "n" is 0, −1 or −2; "m" represents a molar amount of "R" represent per mole of $(M_xAl_yP_z)O_2$ and has a value of from greater than zero (0) to 0.3; "x", "y" and "z" represent the mole fractions of "M", aluminum and phosphorus, respectively, present as tetrahedral oxides, said mole fractions being such that they are within a pentagonal compositional area defined by points A, B, C, D and E, said points A, B, C, D and E having the following values for "x", "y" and "z":

| Point | Mole Fraction x | y | z |
|---|---|---|---|
| A | 0.02 | 0.60 | 0.38 |
| B | 0.02 | 0.38 | 0.60 |
| C | 0.39 | 0.01 | 0.60 |
| D | 0.98 | 0.01 | 0.01 |
| E | 0.39 | 0.60 | 0.01 |

2. Molecular sieves according to claim 1 wherein the mole fractions of "M", aluminum and phosphorus present as tetrahedral oxides are within a hexagonal compositional area defined by points a, b, c, d, e and f, said points a, b, c, d, e and f having the following values for "x", "y" and "z":

| Point | Mole Fraction x | y | z |
|---|---|---|---|
| a | 0.02 | 0.60 | 0.38 |
| b | 0.02 | 0.38 | 0.60 |
| c | 0.39 | 0.01 | 0.60 |
| d | 0.60 | 0.01 | 0.39 |
| e | 0.60 | 0.39 | 0.01 |
| f | 0.39 | 0.60 | 0.01 |

3. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.3 - 7.65 | 12.1 - 11.56 | m - vs |
| 19.5 - 19.95 | 4.55 - 4.46 | m - s |
| 20.9 - 21.3 | 4.25 - 4.17 | m - vs |
| 22.2 - 22.6 | 4.00 - 3.93 | w - vs |
| 25.7 - 26.15 | 3.47 - 3.40 | w - m |

4. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.3 - 9.65 | 9.51 - 9.17 | m - s |
| 20.2 - 20.6 | 4.40 - 4.31 | m - s |
| 20.9 - 21.3 | 4.25 - 4.17 | s - vs |
| 22.0 - 22.5 | 4.04 - 3.95 | m - s |
| 22.5 - 22.9 | 3.95 - 3.92 | m - s |
| 23.0 - 23.4 | 3.87 - 3.80 | m - vs |

5. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 8.6 - 8.9 | 10.3 - 9.93 | vs |
| 13.0 | 6.81 | w |
| 21.9 - 22.2 | 4.06 - 4.00 | w |
| 25.4 | 3.51 | w |
| 27.5 | 3.24 | w |
| 29.7 | 3.01 | w |

6. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 11.3 - 11.6 | 7.83 - 7.63 | m - vs |
| 18.7 - 18.9 | 4.75 - 4.70 | w - s |
| 21.9 - 22.3 | 4.06 - 3.99 | m - vs |
| 26.5 - 27.0 | 3.363 - 3.302 | w - m |
| 29.7 - 30.05 | 3.008 - 2.974 | w - m |

7. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.7 - 7.75 | 11.5 - 11.4 | vs |
| 13.4 | 6.61 | s - vs |
| 15.5 - 15.55 | 5.72 - 5.70 | s |
| 19.65 - 19.7 | 4.52 - 4.51 | w - s |
| 20.5 - 20.6 | 4.33 - 4.31 | vs |
| 31.8 - 32.00 | 2.812 - 2.797 | w - s |

8. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.6 - 9.65 | 9.21 - 9.16 | vs |
| 15.5 - 15.55 | 5.72 - 5.70 | m |
| 16.9 - 17.1 | 5.25 - 5.19 | m |
| 20.15 - 20.25 | 4.41 - 4.39 | m |
| 20.95 - 21.05 | 4.24 - 4.22 | m |
| 31.8 - 32.5 | 2.814 - 2.755 | m |

9. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 13.7 - 14.25 | 6.46 - 6.22 | m - vs |
| 19.55 - 20.0 | 4.54 - 4.44 | w - s |
| 24.05 - 24.5 | 3.70 - 3.63 | m - vs |
| 34.3 - 35.0 | 2.614 - 2.564 | vw - w |
| 42.5 - 43.0 | 2.127 - 2.103 | vw - w |

10. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 8.5 - 8.6 | 10.40 - 10.28 | m - s |
| 20.2 - 20.3 | 4.40 - 4.37 | m |
| 21.9 - 22.1 | 4.06 - 4.02 | w - m |
| 22.6 - 22.7 | 3.93 - 3.92 | vs |
| 31.7 - 31.8 | 2.823 - 2.814 | w - m |

11. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.25 - 9.55 | 9.56 - 9.26 | w - m |
| 12.5 - 12.9 | 7.08 - 6.86 | vs |
| 16.9 - 17.3 | 5.25 - 5.13 | w - m |
| 20.45 - 20.9 | 4.34 - 4.25 | w - m |
| 23.85 - 24.25 | 3.73 - 3.67 | w - m |
| 26.05 - 26.35 | 3.42 - 3.38 | w - m |
| 27.3 - 27.6 | 3.27 - 3.23 | vs |

* as-synthesized form

12. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 13.15 - 13.4 | 6.73 - 6.61 | vs |
| 18.05 - 18.35 | 4.91 - 4.83 | m |
| 18.4 - 18.6 | 4.82 - 4.77 | m |
| 26.55 - 26.7 | 3.36 - 3.34 | m |
| 32.0 - 32.1 | 2.80 - 2.79 | m |

* calcined form

13. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.4 - 9.65 | 9.41 - 9.17 | s - vs |
| 15.9 - 16.2 | 5.57 - 5.47 | vw - m |
| 17.85 - 18.4 | 4.97 - 4.82 | w - s |
| 20.3 - 20.9 | 4.37 - 4.25 | m - vs |
| 24.95 - 25.4 | 3.57 - 3.51 | vw - s |
| 30.3 - 30.8 | 2.95 - 2.90 | w - s |

14. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 10.8 - 11.1 | 8.19 - 7.97 | m |
| 17.2 - 17.4 | 5.16 - 5.10 | s - vs |
| 21.0 - 21.25 | 4.23 - 4.18 | m - s |
| 21.8 - 22.0 | 4.08 - 4.04 | vs |
| 31.8 - 32.2 | 2.814 - 2.788 | m |

15. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.7 - 7.9 | 11.5 - 11.2 | vs |
| 16.2 - 16.6 | 5.47 - 5.34 | w - m |
| 18.9 - 19.3 | 4.70 - 4.60 | m - s |
| 20.6 - 20.8 | 4.31 - 4.27 | w - s |
| 21.8 - 22.0 | 4.08 - 4.04 | m |
| 22.2 - 22.5 | 4.00 - 3.95 | w - m |

16. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 6.1 - 6.3 | 14.49 - 14.03 | vs |
| 15.5 - 15.7 | 5.72 - 5.64 | w - m |
| 18.5 - 18.8 | 4.80 - 4.72 | w - m |
| 23.5 - 23.7 | 3.79 - 3.75 | w - m |
| 26.9 - 27.1 | 3.31 - 3.29 | w - m |

17. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.4 - 9.6 | 9.41 - 9.21 | w - m |
| 13.3 - 13.6 | 6.66 - 6.51 | m - vs |
| 18.0 - 18.4 | 4.93 - 4.82 | m |
| 21.2 - 21.5 | 4.19 - 4.13 | m - s |
| 22.5 - 23.0 | 3.95 - 3.87 | s - vs |
| 30.2 - 30.5 | 2.96 - 2.93 | w - m |

18. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.5 - 7.7 | 11.79 - 11.48 | vw - m |
| 8.0 - 8.1 | 11.05 - 10.94 | s - vs |
| 12.4 - 12.5 | 7.14 - 7.08 | w - vs |
| 13.6 - 13.8 | 6.51 - 6.42 | m - s |
| 14.0 - 14.1 | 6.33 - 6.28 | w - m |
| 27.8 - 28.0 | 3.209 - 3.187 | w - m |

19. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 13.6 - 13.8 | 6.51 - 6.42 | w - m |
| 20.5 - 20.6 | 4.33 - 4.31 | w - m |
| 21.1 - 21.3 | 4.21 - 4.17 | vs |
| 22.1 - 22.3 | 4.02 - 3.99 | m - s |
| 22.8 - 23.0 | 3.90 - 3.86 | m |
| 23.1 - 23.4 | 3.82 - 3.80 | w - m |
| 25.5 - 25.9 | 3.493 - 3.440 | w - m |

20. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.15 - 7.4 | 12.36 - 11.95 | m - vs |
| 12.5 - 12.7 | 7.08 - 6.97 | m - s |
| 21.75 - 21.9 | 4.09 - 4.06 | m - s |
| 24.1 - 24.25 | 3.69 - 3.67 | vs |
| 27.25 - 27.4 | 3.273 - 3.255 | s |
| 30.05 - 30.25 | 2.974 - 2.955 | m - s |

21. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.4 - 9.55 | 9.41 - 9.26 | vs |
| 13.0 - 13.1 | 6.81 - 6.76 | w - m |
| 16.0 - 16.2 | 5.54 - 5.47 | w - m |
| 20.6 - 20.85 | 4.31 - 4.26 | s - vs |
| 24.3 - 24.4 | 3.66 - 3.65 | w - vs |
| 30.7 - 30.95 | 2.912 - 2.889 | w - s |

22. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 7.2 - 8.1 | 12.3 - 10.9 | vs |
| 21.2 - 21.8 | 4.19 - 4.08 | w - m |
| 22.5 - 23.0 | 3.95 - 3.87 | vw - m |
| 26.6 - 27.2 | 3.351 - 3.278 | vw - w |
| 28.5 - 29.0 | 3.132 - 3.079 | vw - w |

23. The crystalline molecular sieves of claims 1 or 2 having a characteristic x-ray powder diffraction pattern which contains at least the d-spacings set forth in the following Table:

| 2θ | d(Å) | Relative Intensity |
|---|---|---|
| 9.4 | 9.41 | vs |
| 15.9 - 16.0 | 5.57 - 5.54 | w - m |
| 20.5 - 20.6 | 4.33 - 4.31 | s |
| 24.5 - 24.7 | 3.63 - 3.60 | w |
| 25.8 - 25.9 | 3.45 - 3.44 | w |
| 30.4 - 30.5 | 2.940 - 2.931 | w |

24. Process for preparing the molecular sieves of claim 1 having crystalline three-dimensional microporous framework structures comprising hydrothermal crystallization preferably at a temperature between 50°C and 250°C and preferably for a reaction time of at least 2 hours until crystals of the molecular sieves of claim 1 are produced from a reaction mixture composition expressed in terms of molar oxide ratios as follows:

$$aR : (M_xAl_yP_z)O_2 : bH_2O$$

wherein "R" is an organic templating agent; "a" is the amount of "R" is preferably an effective amount greater than zero to 6; "M" represents iron and/or titanium, and at least one of cobalt, magnesium, manganese and zinc; "b" has a value of between greater than zero and 500; "x", "y" and "z" represent the mole fractions, respectively, of "M", aluminum and phosphorus in the $(M_xAl_yP_z)O_2$ constituent, and each has a value of at least 0.01, and where "x", "y" and "z" are within a pentagonal compositional area defined by points F, G, H, I and J, said points F, G, H, I and J representing the following values for "x", "y" and "z":

| Point | Mole Fraction x | y | z |
|---|---|---|---|
| F | 0.02 | 0.60 | 0.38 |
| G | 0.02 | 0.38 | 0.60 |
| H | 0.39 | 0.01 | 0.60 |
| I | 0.98 | 0.01 | 0.01 |
| J | 0.39 | 0.60 | 0.01 |

25. Process according to claim 24 wherein the source of phosphorous in the reaction mixture is orthophosphoric acid.

26. Process according to claim 24 wherein the source of phosphorus in the reaction mixture is orthophosphoric acid and the source of aluminum is at least one compound selected from the group of pseudo-boehmite and aluminum alkoxide.

27. Process according to claim 26 wherein the aluminum alkoxide is aluminum isopropoxide.

28. Process according to claim 24 wherein the sources of iron, titanium, cobalt, magnesium, manganese and zinc are selected from the group consisting of chlorides, bromides, iodides, oxides, hydroxides, alkoxides, nitrates, sulfates, acetates and mixtures thereof.

29. Process according to claim 24 wherein the organic templating agent is a quaternary ammonium or quaternary phosphonium compound having the formula

$$R^1_4X^+$$

wherein X is nitrogen or phosphorus and each $R^1$ is an alkyl or aryl group containing from 1 to 8 carbon atoms.

30. Process according to claim 24 wherein the organic templating agent is an amine.

31. Process according to claim 24 wherein the templating agent is selected from the group consisting of tetrapropylammonium ion; tetraethylammonium ion; tripropylamine; triethylamine; triethanolamine; piperidine; cyclohexylamine; 2-methyl pyridine; N,N-dimethylbenzylamine; N,N-diethylethanolamine; choline; N,N'-dimethylpiperazine; 1,4-diazabicyclo-(2,2,2) octane; N-methyldiethanolamine; N-methylethanolamine; N-methylpiperidine; 3-methylpiperidine; N-methylcyclohexylamine; 3-methylpyridine; 4-methylpyridine; quinuclidine; N,N-dimethyl-1,4-diazabicyclo (2,2,2) octane ion; tetramethylammonium ion; tetrabutylammonium ion; tetrapentylammonium ion; di-n-butylamine; neopentylamine; di-n-pentylamine; isopropylamine; t-butylamine; ethylenediamine, pyrrolidine, 2-imidazolidone; di-n-propylamine; and a polymeric quaternary ammonium salt $[(C_{14}H_{32}N_2)(OH)_2]_x$ wherein x is a value of at least 2.

32. Molecular sieves according to one of claims 1 to 23 characterized in that they have been calcined at a temperature sufficiently high to remove at least some of the organic templating agent present in the intracrystalline pore system.

33. Process for separating molecular species from admixture with molecular species having a lesser degree of polarity which comprises contacting said mixture of molecular species with a molecular sieve according to one of claims 1 to 23 having pore diameters large enough to adsorb at least one of the more polar molecular species, said molecular sieve being at least partially activated whereby molecules of the more polar molecular species are selectively adsorbed into the intracrystalline pore system thereof.

34. Process for separating a mixture of molecular species having different kinetic diameters which comprises contacting said mixture with a molecular sieve according to one of claims 1 to 23 having pore diameters large enough to adsorb at least one but not all molecular species of said mixture, said molecular sieve being at least partially activated whereby at least some molecules whose kinetic diameters are sufficiently small can enter the intracrystalline pore system thereof.

35. Process according to claim 33 wherein the more polar molecular species is water.

36. Process for converting a hydrocarbon which comprises contacting said hydrocarbon under hydrocarbon converting conditions with a molecular sieve according to one of claims 1 to 23.

37. Process according to claim 36 wherein the hydrocarbon conversion process is cracking.

38. Process according to claim 36 wherein the hydrocarbon conversion process is hydrocracking.

39. Process according to claim 36 wherein the hydrocarbon conversion process is hydrogenation.

40. Process according to claim 36 wherein the hydrocarbon conversion process is polymerization.

41. Process according to claim 36 wherein the hydrocarbon conversion process is alkylation.

42. Process according to claim 36 wherein the hydrocarbon conversion process is reforming.

43. Process according to claim 36 wherein the hydrocarbon conversion process is hydrotreating.

44. Process according to claim 36 wherein the hydrocarbon conversion process is isomerization.

45. Process according to claim 44 wherein the hydrocarbon conversion process is xylene isomerization.

46. Process according to claim 36 wherein the hydrocarbon conversion process is dehydrocyclization.

**Patentansprüche**

1. Kristalline Molekularsiebe mit dreidimensionalen mikroporösen Gerüstrukturen aus tetraedrischen $MO_2^n$, $AlO_2$ und $PO_2$ Oxideinheiten mit einer empirischen chemischen Zusammensetzung auf wasserfreier Basis ausgedrückt durch die Formel:

$$mR: (M_xAl_yP_z)O_2$$

in der "R" wenigstens ein organisches Templatagens darstellt, das in dem intrakristallinen Porensystem anwesend ist; "M" wenigstens ein Element aus jeder der Gruppen aus: 1) Eisen und Titan, und 2) Kobalt, Magnesium, Mangan und Zink ist; "n" 0, -1 oder -2 ist; "m" die molare Menge von "R" darstellt, die pro Mol an $(M_xAl_yP_z)O_2$ anwesend ist und einen Wert von größer als 0 bis 0,3 hat) "x", "y" und "z" jeweils die Molfraktionen von "M", Aluminium und Phosphor darstellen, die als tetraedrische Oxide anwesend sind, wobei diese Molfraktionen so gewählt sind, daß sie innerhalb eines pentagonalen Zusammensetzungsgebiets liegen, das durch die Punkte A, B, C, D und E definiert wird, wobei diese Punkte A, B, C, D und E die nachfolgenden Werte für "x", "y" und "z" einnehmen:

| Punkt | Mol-Fraktion x | Mol-Fraktion y | Mol-Fraktion z |
|---|---|---|---|
| A | 0,02 | 0,60 | 0,38 |
| B | 0,02 | 0,38 | 0,60 |
| C | 0,39 | 0,01 | 0,60 |
| D | 0,98 | 0,01 | 0,01 |
| E | 0,39 | 0,60 | 0,01 |

2. Molekularsiebe nach Anspruch 1, bei denen die Molfraktionen von "M", Aluminium und Phosphor, die als tetraedrische Oxide vorliegen, innerhalb eines hexagonalen Zusammensetzungsgebiets liegen, das duech die Punkte, a, b, c, d, e und f definiert wird, wobei diese Punkte a, b, c, d, e und f die nachfolgenden Werte für "x", "y" und "z" aufweisen.

| Punkt | Mol-Fraktion x | Mol-Fraktion y | Mol-Fraktion z |
|---|---|---|---|
| a | 0,02 | 0,60 | 0,38 |
| b | 0,02 | 0,38 | 0,60 |
| c | 0,39 | 0,01 | 0,60 |
| d | 0,60 | 0,01 | 0,39 |
| e | 0,60 | 0,39 | 0,01 |
| f | 0,39 | 0,60 | 0,01 |

3. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 7,3 - 7,65 | 12,1 - 11,56 | m-sst |
| 19,5 - 19,95 | 4,55 - 4,46 | m-st |
| 20,9 - 21,3 | 4,25 - 4,17 | m-sst |
| 22,2 - 22,6 | 4,00 - 3,93 | schw-sst |
| 25,7 - 26,15 | 3,47 - 3,40 | schw-m |

4. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,3 - 9,65 | 9,51 - 9,17 | m-st |
| 20,2 - 20,6 | 4,40 - 4,31 | m-st |
| 20,9 - 21,3 | 4,25 - 4,17 | st-sst |
| 22,0 - 22,5 | 4,04 - 3,95 | m-st |
| 22,5 - 22,9 | 3,95 - 3,92 | m-st |
| 23,0 - 23,4 | 3,87 - 3,80 | m-sst |

5. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 8,6 - 8,9 | 10,3 - 9,93 | sst |
| 13,0 | 6,81 | schw |
| 21,9 - 22,2 | 4,06 - 4,00 | schw |
| 25,4 | 3,51 | schw |
| 27,5 | 3,24 | schw |
| 29,7 | 3,01 | schw |

6. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 11,3 - 11,6 | 7,83 - 7,63 | m-sst |
| 18,7 - 18,9 | 4,75 - 4,70 | schw-st |
| 21,9 - 22,3 | 4,06 - 3,99 | m-sst |
| 26,5 - 27,0 | 3,363 - 3,302 | schw-m |
| 29,7 - 30,05 | 3,008 - 2,974 | schw-m |

7. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 7,7 - 7,75 | 11,5 - 11,4 | sst |
| 13,4 | 6,61 | st-sst |
| 15,5 - 15,55 | 5,72 - 5,70 | st |
| 19,65 - 19,7 | 4,52 - 4,51 | schw-st |
| 20,5 - 20,6 | 4,33 - 4,31 | sst |
| 31,8 - 32,00 | 2,812- 2,797 | schw-st |

8. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,6 - 9,65 | 9,21 - 9,16 | sst |
| 15,5 - 15,55 | 5,72 - 5,70 | m |
| 16,9 - 17,1 | 5,25 - 5,19 | m |
| 20,15- 20,25 | 4,41 - 4,39 | m |
| 20,95- 21,05 | 4,24 - 4,22 | m |
| 31,8 - 32,5 | 2,814- 2,755 | m |

9. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 13,7 - 14,25 | 6,46 - 6,22 | m-sst |
| 19,55- 20,0 | 4,54 - 4,44 | schw-st |
| 24,05- 24,5 | 3,70 - 3,63 | m-sst |
| 34,3 - 35,0 | 2,614- 2,564 | sschw-schw |
| 42,5 - 43,0 | 2,127- 2,103 | sschw-schw |

10. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 8,5 - 8,6 | 10,40 - 10,28 | m-st |
| 20,2 - 20,3 | 4,40 - 4,37 | m |
| 21,9 - 22,1 | 4,06 - 4,02 | schw-m |
| 22,6 - 22,7 | 3,93 - 3,92 | sst |
| 31,7 - 31,8 | 2,823- 2,814 | schw-m |

11. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,25 - 9,55 | 9,56 - 9,26 | schw-m |
| 12,5 -12,9 | 7,08 - 6,86 | sst |
| 16,9 -17,3 | 5,25 - 5,13 | schw-m |
| 20,45 -20,9 | 4,34 - 4,25 | schw-m |
| 23,85 -24,25 | 3,73 - 3,67 | schw-m |
| 26,05 -26,35 | 3,42 - 3,38 | schw-m |
| 27,3 -27,6 | 3,27 - 3,23 | sst |

*) wie synthetisiert

12. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 13,15 - 13,4 | 6,73 - 6,61 | sst |
| 18,05 - 18,35 | 4,91 - 4,83 | m |
| 18,4 - 18,6 | 4,82 - 4,77 | m |
| 26,55 - 26,7 | 3,36 - 3,34 | m |
| 32,0 - 32,1 | 2,80 - 2,79 | m |

*) calcinierte Form

13. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,4 - 9,65 | 9,41 - 9,17 | st-sst |
| 15,9 -16,2 | 5,57 - 5,47 | sschw-m |
| 17,85-18,4 | 4,97 - 4,82 | schw-st |
| 20,3 -20,9 | 4,37 - 4,25 | m-sst |
| 24,95-25,4 | 3,57 - 3,51 | sschw-st |
| 30,3 -30,8 | 2,95 - 2,90 | schw-st |

14. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 10,8 - 11.1 | 8,19 - 7,97 | m |
| 17,2 - 17,4 | 5,16 - 5,10 | st-sst |
| 21,0 - 21,25 | 4,23 - 4,18 | m-st |
| 21,8 - 22,0 | 4,08 - 4,04 | sst |
| 31,8 - 32,2 | 2,814-2,788 | m |

15. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 7,7 - 7,9 | 11,5 - 11,2 | sst |
| 16,2 - 16,6 | 5,47 - 5,34 | schw-m |
| 18,9 - 19,3 | 4,70 - 4,60 | m-st |
| 20,6 - 20,8 | 4,31 - 4,27 | schw-st |
| 21,8 - 22,0 | 4,08 - 4,04 | m |
| 22,2 - 22,5 | 4,00 - 3,95 | schw-m |

16. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 6,1 - 6,3 | 14,49 - 14,03 | sst |
| 15,5 - 15,7 | 5,72 - 5,64 | schw-m |
| 18,5 - 18,8 | 4,80 - 4,72 | schw-m |
| 23,5 - 23,7 | 3,79 - 3,75 | schw-m |
| 26,9 - 27,1 | 3,31 - 3,29 | schw-m |

17. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,4 - 9,6 | 9,41 - 9,21 | schw-m |
| 13,3 - 13,6 | 6,66 - 6,51 | m-sst |
| 18,0 - 18,4 | 4,93 - 4,82 | m |
| 21,2 - 21,5 | 4,19 - 4,13 | m-st |
| 22,5 - 23,0 | 3,95 - 3,87 | st-sst |
| 30,2 - 30,5 | 2,96 - 2,93 | schw-m |

18. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 7,5 - 7,7 | 11,79 - 11,48 | sschw-m |
| 8,0 - 8,1 | 11,05 - 10,94 | st-sst |
| 12,4 - 12,5 | 7,14 - 7,08 | schw-sst |
| 13,6 - 13,8 | 6,51 - 6,42 | m-st |
| 14,0 - 14,1 | 6,33 - 6,28 | schw-m |
| 27,8 - 28,0 | 3,209- 3,187 | schw-m |

19. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 13,6 - 13,8 | 6,51 - 6,42 | schw-m |
| 20,5 - 20,6 | 4,33 - 4,31 | schw-m |
| 21,1 - 21,3 | 4,21 - 4,17 | sst |
| 22,1 - 22,3 | 4,02 - 3,99 | m-st |
| 22,8 - 23,0 | 3,90 - 3,86 | m |
| 23,1 - 23,4 | 3,82 - 3,80 | schw-m |
| 25,5 - 25,9 | 3,493-3,440 | schw-m |

20. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 7,15 - 7,4 | 12,36 - 11,95 | m-sst |
| 12,5 - 12,7 | 7,08 - 6,97 | m-st |
| 21,75 - 21,9 | 4,09 - 4,06 | m-st |
| 24,1 - 24,25 | 3,69 - 3,67 | sst |
| 27,25 - 27,4 | 3,273- 3,255 | st |
| 30,05 - 30,25 | 2,974- 2,955 | m-st |

21. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2θ | d (Å) | relative Intensität |
|---|---|---|
| 9,4 - 9,55 | 9,41 - 9,26 | sst |
| 13,0 - 13,1 | 6,81 - 6,76 | schw-m |
| 16,0 - 16,2 | 5,54 - 5,47 | schw-m |
| 20,6 - 20,85 | 4,31 - 4,26 | st-sst |
| 24,3 - 24,4 | 3,66 - 3,65 | schw-sst |
| 30,7 - 30,95 | 2,912- 2,889 | schw-st |

22. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2Θ | d (Å) | relative Intensität |
|---|---|---|
| 7,2 - 8,1 | 12,3 - 10,9 | sst |
| 21,2 - 21,8 | 4,19 - 4,08 | schw-m |
| 22,5 - 23,0 | 3,95 - 3,87 | sschw-m |
| 26,6 - 27,2 | 3,351- 3,278 | sschw-schw |
| 28,5 - 29,0 | 3,132- 3,079 | sschw-schw |

23. Kristalline Molekularsiebe gemäß Anspruch 1 oder 2 mit einem charakteristischen Röntgenstrahlpulverbeugungsmuster, das wenigstens die in der nachfolgenden Tabelle wiedergegebenen d-Werte enthält:

| 2Θ | d (Å) | relative Intensität |
|---|---|---|
| 9,4 | 9,41 | sst |
| 15,9 - 16,0 | 5,57 - 5,54 | schw-m |
| 20,5 - 20,6 | 4,33 - 4,31 | st |
| 24,5 - 24,7 | 3,63 - 3,60 | schw |
| 25,8 - 25,9 | 3,45 - 3,44 | schw |
| 30,4 - 30,5 | 2,940- 2,931 | schw |

24. Verfahren zur Herstellung der Molekularsiebe gemäß Anspruch 1, die kristalline dreidimensionale mikroporöse Gerüststrukturen haben, umfassend die hydrothermische Kristallisierung, vorzugsweise bei einer Temperatur zwischen 50°C und 250°C und vorzugsweise für eine Reaktionsdauer von wenigstens 2 Stunden, bis Kristalle der Molekularsiebe gemäß Anspruch 1 gebildet werden, ausgehend von einer Reaktionsmischung mit einer Zusammensetzung ausgedrückt zahlenmäßig durch die nachfolgenden molaren Verhältnisse der Oxide:

$$aR: (M_xAl_yP_z)O_2:bH_2O$$

worin "R" ein organisches Templatagens ist, "a" die Menge an "R" darstellt und vorzugsweise eine effektive Menge größert als Null bis 6 darstellt, "M" Eisen und/oder Titan darstellt und wenigstens ein Element ausgewählt aus Cobalt, Magnesium, Mangan und Zink, "b" einen Wert von größer als Null bis 500 hat, "x", "y" und "z" jeweils die Molfraktionen an "M", Aluminium und Phosphor in dem $(M_xAl_yP_z)O_2$ Bestandteil darstellen und jeweils einen Wert von wenigstens 0,01 haben, wobei "x", "y" und "z" innerhalb eines pentagonalen Zusammensetzungsbereichs liegen, der durch die Punkte F. G. H, I und J definiert ist, wobei diese Punkte "f, G, H, I und J die nachfolgenden Werte für "x", "y" und "z" darstellen:

| Punkt | Mol-Fraktion x | Mol-Fraktion y | Mol-Fraktion z |
|---|---|---|---|
| F | 0,02 | 0,60 | 0,38 |
| G | 0,02 | 0,38 | 0,60 |
| H | 0,39 | 0,01 | 0,60 |
| I | 0,98 | 0,01 | 0,01 |
| J | 0,39 | 0,60 | 0,01 |

25. Verfahren nach Anspruch 24, bei dem die Quelle für Phosphor in der Reaktionsmischung Orthophosphorsäure ist.

26. Verfahren nach Anspruch 24, bei dem die Quelle für Phosphor in der Reaktionsmischung Orthophosphorsäure ist und die Quelle für Aluminium wenigstens eine Verbindung ausgewählt aus der Gruppe bestehend aus pseudo-Boehmit und Aluminiumalkoxid ist.

27. Verfahren nach Anspruch 26, bei dem das Aluminium alkoxid Aluminimisopropoxid ist.

28. Verfahren nach Anspruch 24, bei dem die Quellen für Eisen, Titan, Cobalt, Mangnesium, Mangan und Zink ausgewählt werden aus der Gruppe bestehend aus den Chloriden, Bromiden, Jodiden, Oxiden, Hydroxyiden, Alkoxiden, Nitraten, Sulfaten, Acetaten und deren Mischungen.

29. Verfahren nach Anspruch 24, bei dem das organische Templatagens eine quaternäre Ammonium- oder quaternäre Phosphoniumverbindung mit der Formel ist

$$R^1_4X^+$$

in der X Stickstoff oder Phosphor ist und $R^1$ jeweils eine Alkyl- oder Arylgruppe mit 1 bis 8 Kohlenstoffatomen darstellt.

30. Verfahren nach Anspruch 24, bei dem das organische Templatagens ein Amin ist.

31. Verfahren nach Anspruch 24, bei dem ds Templatagens ausgewählt wird aus der Gruppe bestehend aus dem Tetrapropylammoniumion, Tetraethylammoniumion, Tripropylamin, Triethylamin, Triethanolamin, Piperidin, Cyclohexylamin, 2-Methylpyridin, N,N-Dimethylbenzylamin, N,N-Diethylethanolamin, Chlorin, N,N-Dimethylpiperazin, 1,4-Diazabicyclo(2,2,2)octan, N-Methyldiethanolamin, N-Methylethanolamin, N-Methylpiperidin, 3-Methylpiperidin, N-Methylcyclohexylamin, 3-Methylpyridin, 4-Methylpyridin, Chinuclidin, N,N-Dimethyl-1,4-diazabicyclo(2,2,2)octanion, Tetramethylammoniumion, Tetrabutylammoniumion, Tetrapentylammoniumion, Di-n-Butylamin, Neopentylamin, Di-n-pentylamin, Isopropylamin, t-Butylamin, Ethylendiamin, Pyrrolidin, 2-Imidazolidon, Di-n-propylamin und einem polymeren quaternären Ammoniumsalz $[(C_{14}H_{32}N_2)]_x$ in dem x einen Wert von wenigstens 2 hat.

32. Molekularsiebe gemäß einem der Ansprüche 1 bis 23, dadurch gekennzeichnet, daß sie bei einer Temperatur calciniert wurden, die ausreichend hoch ist, um wenigstens einen Teil des im intrakristallinen Porensystem anwesenden organischen Templatagens zu entferenen.

33. Verfahren zur Trennung molekularer Spezies aus einem Gemisch mit molekularen Spezies, die einen geringeren Polaritätsgrad aufweisen, umfassend das in Kontakt bringen dieses Gemischs molekularere Spezies mit einem Molekularsieb gemäß einem der Ansprüche 1 bis 23, das Porendurchmesser aufweist, die weit genug sind wenigstens einer der polareren molekularen Spezies zu adsorbieren, wobei dieses Molekularsieb wenigstens teilweise aktiviert wurde, wobei Moleküle der polareren molkularen Spezies selektiv in dessen intrakristallines Porensystem adsorbiert werden.

34. Verfahren zur Trennung eines Gemischs molekularer Spezies mit unterschiedlichen kinetischen Durchmessern umfassend das in Kontakt bringen dieses Gemischs mit einem Molekularsieb gemäß einem der Ansprüche 1 bis 23, das Porendurchmesser aufweist, die groß genung sind, wenigstens eine jedoch nicht alle molekularen Spezies aus dieser Mischung zu adsorbieren, wobei dieses Molekularsieb wenigstens teilweise aktiviert ist, wobei wenigstens einige Moleküle, deren kinetische Durchmesser ausreichend klein sind in dessen intrakristallines Porensystem eintreten können.

35. Verfahren nach Anspruch 33 bei dem die polarere molekulare spezies Wasser ist.

36. Verfahren zur Umwandlung eines Kohlenwasserstoffs umfassend das in Kontakt bringen dieses Kohlenwassestoffs unter Kohlenwasserstoffumwandlungsbedingungen mit einem Molekularsieb gemäß einem der Ansprüche 1 bis 23.

37. Verfanren nach Anspruch 36, bei dem der Kohlenwasserstoff-Umwandlungs-Prozeß Cracking ist.

38. Verfahren nach Anspruch 36, bei dem der Kohlenwasserstoff-Umwandlungs-Prozeß Hydrocracking ist.

39. Verfahren nach Anspruch 36, bei dem der Kohlenwasserstoff-Umwandlungs-Prozeß eine Hydrogenierung ist.

40. Verfahren nach Anspruch 36, bei dem der Kohlenwasserstoff-Umwandlungs-Prozeß eine Polymerisation ist.

41. Verfahren nach Anspruch 36, bei dem der Kohlenwasserstoff-Umwandlungs-Prozeß eine Alkylierung ist.

42. Verfahren nach Anspruch 36, bei dem der Kohlenwasserstoff-Umwandlungs-Prozeß Reformieren ist.

43. Verfahren nach Anspruch 36, bei dem der Kohlenwasserstoff-Umwandlungs-Prozeß eine Hydrobehandlung ist.

44. Verfahren nach Anspruch 36, bei dem der Kohlenwasserstoff-Umwandlungs-Prozeß eine Isomerisierung ist.

45. Verfahren nach Anspruch 36, bei dem der Kohlenwasserstoff-Umwandlungs-Prozeß eine Xylenisomerisierung ist.

46. Verfahren nach Anspruch 36, bei dem der Kohlenwasserstoff-Umwandlungs-Prozeß eine Dehydrocyclosierung ist.

**Revendications**

1. Tamis moléculaires cristallins ayant des ossatures structurales microporeuses tridimensionnelles formées de motifs d'oxydes tétraédriques $MO_2^n$, $AlO_2$ et $PO_2$ dont la composition chimique, sur base anhydre, s'exprime par la formule brute:

$$mR: (M_xAl_yP_z)O_2$$

dans laquelle "R" représente un moins un agent organique d'orientation structurale présent dans la système de pores intracristallins; "M" représente au moins un élément choisi dans chacune des classes comprenant: 1) le fer et le titane; et 2) le cobalt, le magnésium, le manganèse et le zinc; "n" a la valeur 0, -1 ou -2, "m" représente une quantité molaire de "R" par mole de $(M_xAl_yP_z)O_2$ et a une valeur allant de plus de zéro (O) à 0,3; "x", "y" et "z" représentent les fractions molaires respectives de "M", d'aluminum et de phosphore présentes à l'état d'oxydes tétraédriques, ces fractions molaires étant choisies de manière qu'elles rentrent dans l'aire pentagonale de composition délimitée par les points A, B, C, D et E, lesdits points A, B, C, D et E ayant les valeurs suivants pour "x", "y" et "z":

| | Fraction molaire | | |
|---|---|---|---|
| Point | x | y | z |
| A | 0,02 | 0,60 | 0,38 |
| B | 0,02 | 0,38 | 0,60 |
| C | 0,39 | 0,01 | 0,60 |
| D | 0,98 | 0,01 | 0,01 |
| E | 0,39 | 0,60 | 0,01 |

2. Tamis moléculaires suivant la revendication 1, dans lesquels les fractions molaires de "M" d'aluminium et de phosphore présentes à l'état d'oxydes tétraédriques rentrent dans une aire hexagonale de composition délimitée par les points a, b, c, d, e et f, lesdits points a, b, c, d, e et f ayant les valeurs suivantes pour "x", "y" et "z":

| | Fraction molaire | | |
|---|---|---|---|
| Point | x | y | z |
| a | 0,02 | 0,60 | 0,38 |
| b | 0,02 | 0,38 | 0,60 |
| c | 0,39 | 0,01 | 0,60 |
| d | 0,60 | 0,01 | 0,39 |
| e | 0,60 | 0,39 | 0,01 |
| f | 0,39 | 0,60 | 0,01 |

3. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,3 - 7,65 | 12,1 - 11,56 | m - vs |
| 19,5 - 19,95 | 4,55 - 4,46 | m - s |
| 20,9 - 21,3 | 4,25 - 4,17 | m - vs |
| 22,2 - 22,6 | 4,00 - 3,93 | w - vs |
| 25,7 - 26,15 | 3,47 - 3,40 | w - m |

4. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,3 - 9,65 | 9,51 - 9,17 | m - s |
| 20,2 - 20,6 | 4,40 - 4,31 | m - s |
| 20,9 - 21,3 | 4,25 - 4,17 | s - vs |
| 22,0 - 22,5 | 4,04 - 3,95 | m - s |
| 22,5 - 22,9 | 3,95 - 3,92 | m - s |
| 23,0 - 23,4 | 3,87 - 3,80 | m - vs |

5. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 8,6 - 8,9 | 10,3 - 9,93 | vs |
| 13,0 | 6,81 | w |
| 21,9 - 22,2 | 4,06 - 4,00 | w |
| 25,4 | 3,51 | w |
| 27,5 | 3,24 | w |
| 29,7 | 3,01 | w |

6. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 11,3 - 11,6 | 7,83 - 7,63 | m - vs |
| 18,7 - 18,9 | 4,75 - 4,70 | w - s |
| 21,9 - 22,3 | 4,06 - 3,99 | m - vs |
| 26,5 - 27,0 | 3,363 - 3,302 | w - m |
| 29,7 - 30,05 | 3,008 - 2,974 | w - m |

7. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,7 - 7,75 | 11,5 - 11,4 | vs |
| 13,4 | 6,61 | s - vs |
| 15,5 - 15,55 | 5,72 - 5,70 | s |
| 19,65 - 19,7 | 4,52 - 4,51 | w - s |
| 20,5 - 20,6 | 4,33 - 4,31 | vs |
| 31,8 - 32,00 | 2,812 - 2,797 | w - s |

8. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,6 - 9,65 | 9,21 - 9,16 | vs |
| 15,5 - 15,55 | 5,72 - 5,70 | m |
| 16,9 - 17,1 | 5,25 - 5,19 | m |
| 20,15 - 20,25 | 4,41 - 4,39 | m |
| 20,95 - 21,05 | 4,24 - 4,22 | m |
| 31,8 - 32,5 | 2,814 - 2,755 | m |

9. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 13,7 - 14,25 | 6,46 - 6,22 | m - vs |
| 19,55 - 20,0 | 4,54 - 4,44 | w - s |
| 24,05 - 24,5 | 3,70 - 3,63 | m - vs |
| 34,3 - 35,0 | 2,614 - 2,564 | vw - w |
| 42,5 - 43,0 | 2,127 - 2,103 | vw - w |

10. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 8,5 - 8,6 | 10,40 - 10,28 | m - s |
| 20,2 - 20,3 | 4,40 - 4,37 | m |
| 21,9 - 22,1 | 4,06 - 4,02 | w - m |
| 22,6 - 22,7 | 3,93 - 3,92 | vs |
| 31,7 - 31,8 | 2,823 - 2,814 | w - m |

11. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,25 - 9,55 | 9,56 - 9,26 | w - m |
| 12,5 - 12,9 | 7,08 - 6,86 | vs |
| 16,9 - 17,3 | 5,25 - 5,13 | w - m |
| 20,45 - 20,9 | 4,34 - 4,25 | w - m |
| 23,85 - 24,25 | 3,73 - 3,67 | w - m |
| 26,05 - 26,35 | 3,42 - 3,38 | w - m |
| 27,3 - 27,6 | 3.27 - 3.23 | vs |

12. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 13,15 - 13,4 | 6,73 - 6,61 | vs |
| 18,05 - 18,35 | 4,91 - 4,83 | m |
| 18,4 - 18,6 | 4,82 - 4,77 | m |
| 26,55 - 26,7 | 3,36 - 3,34 | m |
| 32,0 - 32,1 | 2,80 - 2,79 | m |

* Forme calcinée

13. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,4 - 9,65 | 9,41 - 9,17 | s - vs |
| 15,9 - 16,2 | 5,57 - 5,47 | vw - m |
| 17,85 - 18,4 | 4,97 - 4,82 | w - s |
| 20,3 - 20,9 | 4,37 - 4,25 | m - vs |
| 24,95 - 25,4 | 3,57 - 3,51 | vw - s |
| 30,3 - 30,8 | 2,95 - 2,90 | w - s |

14. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 10,8 - 11,1 | 8,19 - 7,97 | m |
| 17,2 - 17,4 | 5,16 - 5,10 | s - vs |
| 21,0 - 21,25 | 4,23 - 4,18 | m - s |
| 21,8 - 22,0 | 4,08 - 4,04 | vs |
| 31,8 - 32,2 | 2,814 - 2,788 | m |

15. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,7 - 7,9 | 11,5 - 11,2 | vs |
| 16,2 - 16,6 | 5,47 - 5,34 | w - m |
| 18,9 - 19,3 | 4,70 - 4,60 | m - s |
| 20,6 - 20,8 | 4,31 - 4,27 | w - s |
| 21,8 - 22,0 | 4,08 - 4,04 | m |
| 22,2 - 22,5 | 4,00 - 3,95 | w - m |

16. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 6,1 - 6,3 | 14,49 - 14,03 | vs |
| 15,5 - 15,7 | 5,72 - 5,64 | w - m |
| 18,5 - 18,8 | 4,80 - 4,72 | w - m |
| 23,5 - 23,7 | 3,79 - 3,75 | w - m |
| 26,9 - 27,1 | 3,31 - 3,29 | w - m |

17. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,4 - 9,6 | 9,41 - 9,21 | w - m |
| 13,3 - 13,6 | 6,66 - 6,51 | m - vs |
| 18,0 - 18,4 | 4,93 - 4,82 | m |
| 21,2 - 21,5 | 4,19 - 4,13 | m - s |
| 22,5 - 23,0 | 3,95 - 3,87 | s - vs |
| 30,2 - 30,5 | 2,96 - 2,93 | w - m |

18. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,5 - 7,7 | 11,79 - 11,48 | vw - m |
| 8,0 - 8,1 | 11,05 - 10,94 | s - vs |
| 12,4 - 12,5 | 7,14 - 7,08 | w - vs |
| 13,6 - 13,8 | 6,51 - 6,42 | m - s |
| 14,0 - 14,1 | 6,33 - 6,28 | w - m |
| 27,8 - 28,0 | 3,209 - 3,187 | w - m |

19. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 13,6 - 13,8 | 6,51 - 6,42 | w - m |
| 20,5 - 20,6 | 4,33 - 4,31 | w - m |
| 21,1 - 21,3 | 4,21 - 4,17 | vs |
| 22,1 - 22,3 | 4,02 - 3,99 | m - s |
| 22,8 - 23,0 | 3,90 - 3,86 | m |
| 23,1 - 23,4 | 3,82 - 3,80 | w - m |
| 25,5 - 25,9 | 3,493 - 3,440 | w - m |

20. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,15 - 7,4 | 12,36 - 11,95 | m - vs |
| 12,5 - 12,7 | 7,08 - 6,97 | m - s |
| 21,75 - 21,9 | 4,09 - 4,06 | m - s |
| 24,1 - 24,25 | 3,69 - 3,67 | vs |
| 27,25 - 27,4 | 3,273 - 3,255 | s |
| 30,05 - 30,25 | 2,974 - 2,955 | m - s |

21. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,4 - 9,55 | 9,41 - 9,26 | vs |
| 13,0 - 13,1 | 6,81 - 6,76 | w - m |
| 16,0 - 16,2 | 5,54 - 5,47 | w - m |
| 20,6 - 20,85 | 4,31 - 4,26 | s - vs |
| 24,3 - 24,4 | 3,66 - 3,65 | w - vs |
| 30,7 - 30,95 | 2,912 - 2,889 | w - s |

22. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 7,2 - 8,1 | 12,3 - 10,9 | vs |
| 21,2 - 21,8 | 4,19 - 4,08 | w - m |
| 22,5 - 23,0 | 3,95 - 3,87 | vw - m |
| 26,6 - 27,2 | 3,351 - 3,278 | vw - w |
| 28,5 - 29,0 | 3,132 - 3,079 | vw - w |

23. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d(Å) | Intensité relative |
|---|---|---|
| 9,4 | 9,41 | vs |
| 15,9 - 16,0 | 5,57 - 5,54 | w - m |
| 20,5 - 20,6 | 4,33 - 4,31 | s |
| 24,5 - 24,7 | 3,63 - 3,60 | w |
| 25,8 - 25,9 | 3,45 - 3,44 | w |
| 30,4 - 30,5 | 2,940 - 2,931 | w |

24. Procédé de préparation de tamis moléculaires suivant la revendication 1 présentant des ossatures structurales microporeuses tridimensionnelles cristallines, qui consiste à effectuer la cristallisation hydrothermique, de préférence à une température comprise entre 50°C et 250°C et de préférence pendant une durée de réaction d'au moins 2 heures, jusqu'à ce que des cristaux des tamis moléculaires suivant la

revendication 1 aient été produits à partir d'un mélange réactionnel dont la composition, exprimée en termes de rapports molaires des oxydes, répond à la formule:

$$aR: (M_xAl_yP_z)O_2:bH_2O$$

dans laquelle "R" est un agent organique d'orientation structurale; "a" est la quantité de "R" et est de préférence une quantité efficace allant d'une valeur de plus de zéro à 6; "M" représente le fer et/ou le titane et au moins l'un des métaux cobalt, magnésium, manganèse et zinc; "b" a une valeur allant de plus de zéro à 500; "x", "y" et "z" représentent les fractions molaires respectives de "M", d'aluminum et de phosphor dans le constituant $(M_xAl_yP_z)O_2$ et chacun de ces paramètres a une valeur d'au moins 0,01, et "x", "y" et "z" rentrent dans une aire pentagonale de composition délimitée par les points F, G, H, I et J, lesdits points F, G, H, I et J représentant les valeurs suivantes pour "x", "y" et "z":

| Point | Fraction molaire x | y | z |
|---|---|---|---|
| F | 0,02 | 0,60 | 0,38 |
| G | 0,02 | 0,38 | 0,60 |
| H | 0,39 | 0,01 | 0,60 |
| I | 0,98 | 0,01 | 0,01 |
| J | 0,39 | 0,60 | 0,01 |

25. Procédé suivant la revendication 24, dans lequel la source de phosphore dans le mélange réactionnel est l'acide ortho-phosphorique.

26. Procédé suivant la revendication 24, dans lequel la source de phosphore du mélange réactionnel est l'acide ortho-phosphorique et la source d'aluminium est au moins un composé choisi entre la pseudoboéhmite et un alcoolate d'aluminium.

27. Procédé suivant la revendication 26, dans lequel l'alcoolate d'aluminium est l'isopropylate d'aluminium.

28. Procédé suivant la revendication 24, dans lequel les sources de fer, titane, cobalt, magnésium, manganèse et zinc sont choisies dans le groupe comprenant des chlorures, bromures, iodures, oxydes, hydroxydes, alcoolates, nitrates, sulfates, acétates et leurs mélanges.

29. Procédé suivant la revendication 24, dans lequel l'agent organique d'orientation structurale est un composé d'ammonium quaternaire ou de phosphonium quaternaire répondant à la formule

$$R^1_4X^+$$

dans laquelle X est l'azote ou le phosphore et chaque $R^1$ représente un groupe alkyle ou aryle contenant 1 à 8 atomes de carbone.

30. Procédé suivant la revendication 24, dans lequel l'agent organique d'orientation structurale est une amine.

31. Procédé suivant la revendication 24, dans lequel l'agent d'orientation structurale est choisi dans le groupe comprenant l'ion tétrapropylammonium; l'ion tétraéthylammonium; la tripropylamine; la triéthylamine; la triéthanolamine; la pipéridine; la cyclohexylamine; la 2-méthylpyridine; la N,N-diméthylbenzylamine; la N,N-diéthyléthanolamine; la choline; la N,N'-diméthylpipérazine; le 1,4-diazabicyclo-(2,2,2)octane; la N-méthyldiéthanolamine; la N-méthyléthanolamine; la N-méthylpipéridine; la 3-méthylpipéridine; la N-méthylcyclohexylamine; la 3-méthylpyridine; la 4-méthylpyridine; la quinuclidine; l'ion N,N-diméthyl-1,4-diazabicyclo(2,2,2)octane; l'ion tétraméthylammonium; l'ion tétrabutylammonium; l'ion tétrapentylammonium; la di-n-butylamine; la néopentylamine; la di-n-pentylamine; l'isopropylamine; la tertio-butylamine; l'éthylènediamine; la pyrrolidine; la 2-imidazolidone; la di-n-propylamine et un sel d'ammonium quaternaire polymérique de formule $[(C_{14}H_{32}N_2)(OH)_2]_x$ dans laquelle x a une valeur d'au moins 2.

32. Tamis moléculaires suivant l'une des revendications 1 à 23, caractérisés en ce qu'ils ont été calcinés à une température suffisamment haut pour éliminer au moins une partie de l'agent organique d'orientation structurale présent dans le système de pores intracristallins.

33. Procédé de séparation d'espèces moléculaires d'un mélange avec des espèces moléculaires ayant un plus faible degré de polairité, qui consiste à faire entrer ledit mélange d'espèces moléculaires en contact

avec un tamis moléculaire suivant l'une des revendications 1 à 23, ayant des diamètres de pores suffisamment grands pour adsorber au moins l'une des espèces moléculaires les plus polaires, ledit tamis moléculaire étant au moins partiellement activé de mainère que des molécules de l'espèce moléculaire la plus polaire soient sélectivement adsorbées dans son système de pores intracristallins.

34. Procédé pour séparer un mélange d'espèces moléculaires ayant des diamètres cinétiques différents, qui consiste à faire entrer ledit mélange en contact avec un tamis moléculaire suivant l'une quelconque des revendications 1 à 23, ayant des diamètres de pores suffisamment grands pour adsorber au moins l'une, mais non la totalité, des espèces moléculaires dudit mélange, ledit tamis moléculaire étant au moins partiellement activé de manière qu'au moins certaines molécules dont les diamètres cinétiques sont suffisamment petits puissant pénétrer dans son système de pores intracristallins.

35. Procédé suivant la revendication 33, dans lequel lequel l'espèce moléculaire plus polaire est l'eau.

36. Procédé de transformation d'un hydrocarbure, qui consiste à faire entrer ledit hydrocarbure en contact, dans des conditions de transformation de cet hydrocarbure, avec un tamis moléculaire suivant l'une des revendications 1 à 23.

37. Procédé suivant la revendication 36, dans lequel l'opération de transformation de l'hydrocarbure est un craquage.

38. Procédé suivant la revendication 36, dans lequel l'opération de transformation de l'hydrocarbure est un hydrocraquage.

39. Procédé suivant la revendication 36, dans lequel l'opération de transformation de l'hydrocarbure est une hydrogénation.

40. Procédé suivant la revendication 36, dans lequel l'opération de transformation de l'hydrocarbure est une polymérisation.

41. Procédé suivant la revendication 36, dans lequel l'opération de transformation de l'hydrocarbure est une alkylation.

42. Procédé suivant la revendication 36, dans lequel l'opération de transformation de l'hydrocarbure est un reformage.

43. Procédé suivant la revendication 36, dans lequel l'opération de transformation de l'hydrocarbure est un hydrotraitement.

44. Procédé suivant la revendication 36, dans lequel l'opération de transformation de l'hydrocarbure est une isomérisation.

45. Procédé suivant la revendication 44, dans leque l'opération de transformation de l'hydrocarbure est l'isomérisation de xylènes.

46. Procédé suivant la revendication 36, dans lequel l'opération de transformation de l'hydrocarbure est une déshydrocyclisation.

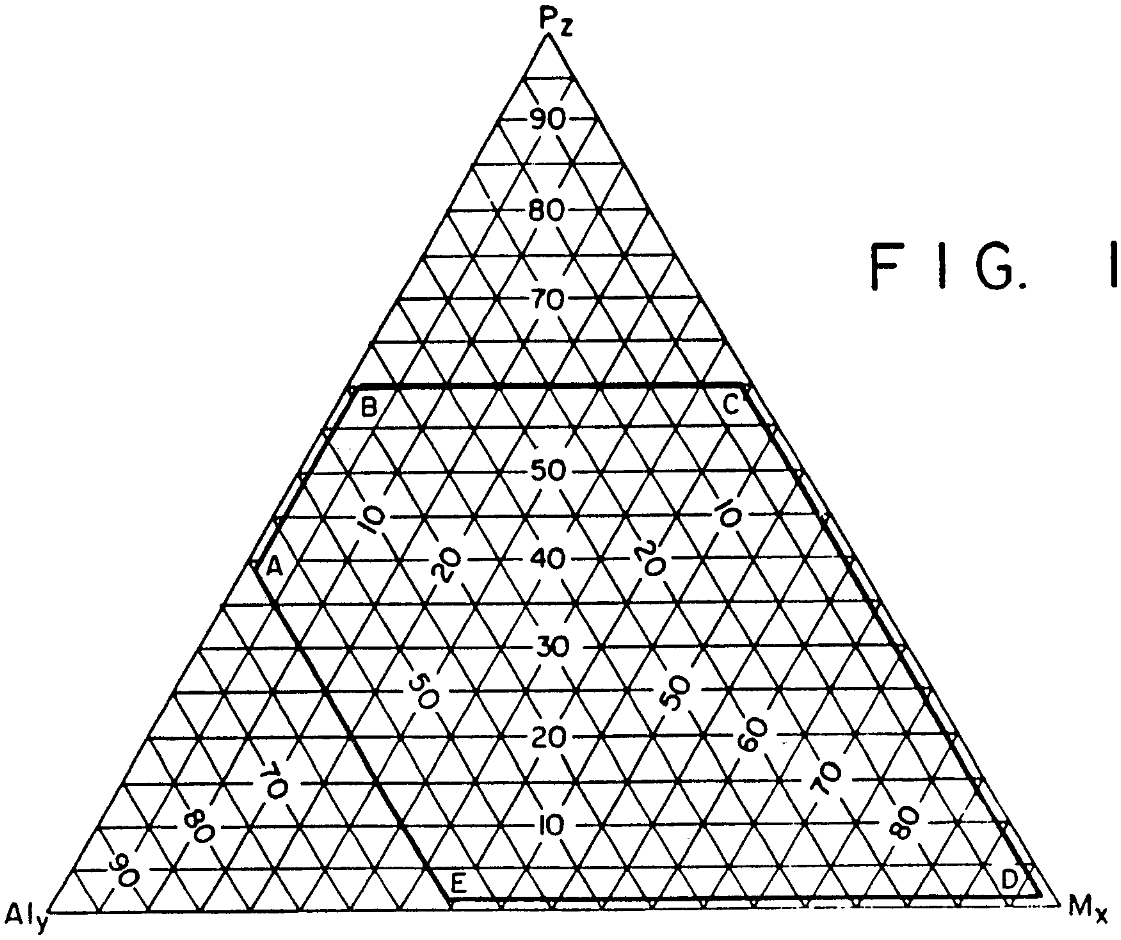
Pz
90
80
70
50
40
30
20
10
B
C
A
D
E
10
20
50
70
80
90
10
20
50
60
70
80
Aly
Mx

FIG. 1

FIG. 2

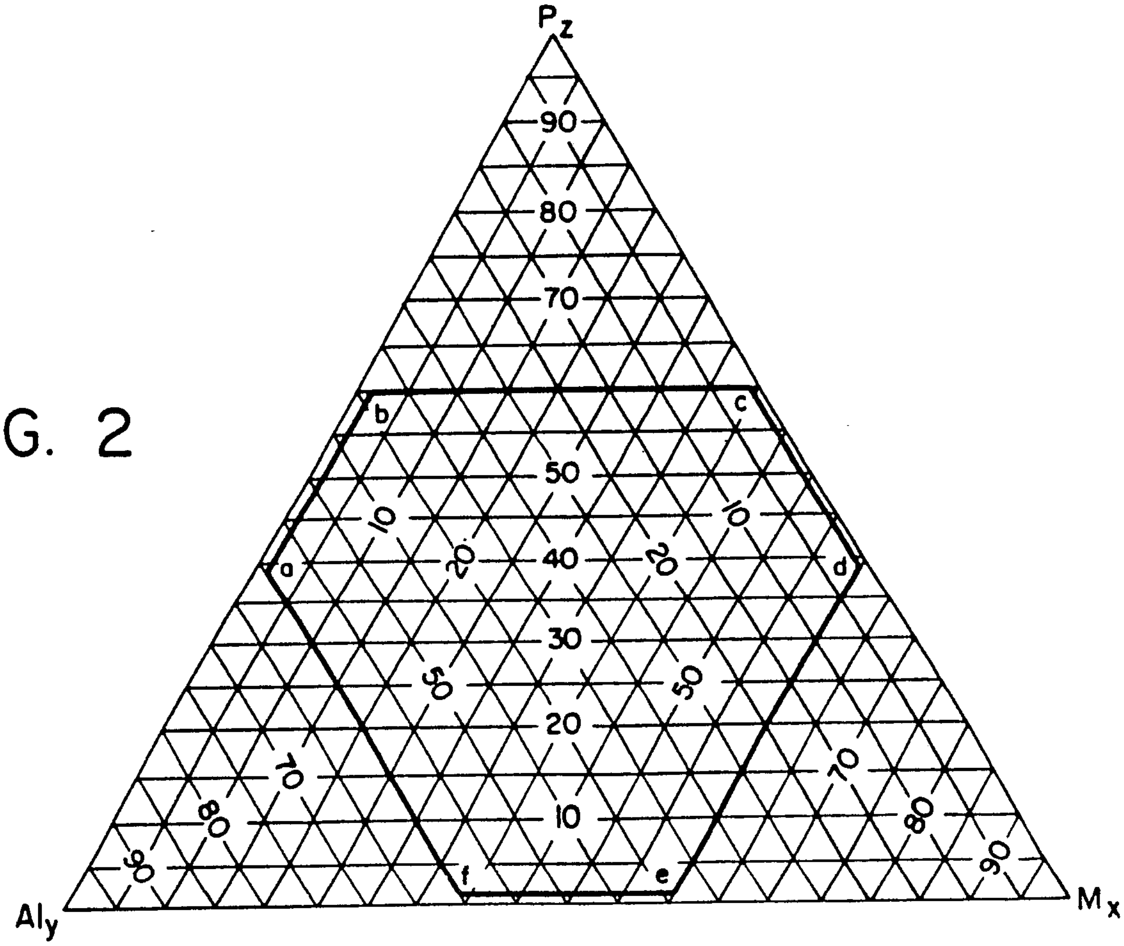

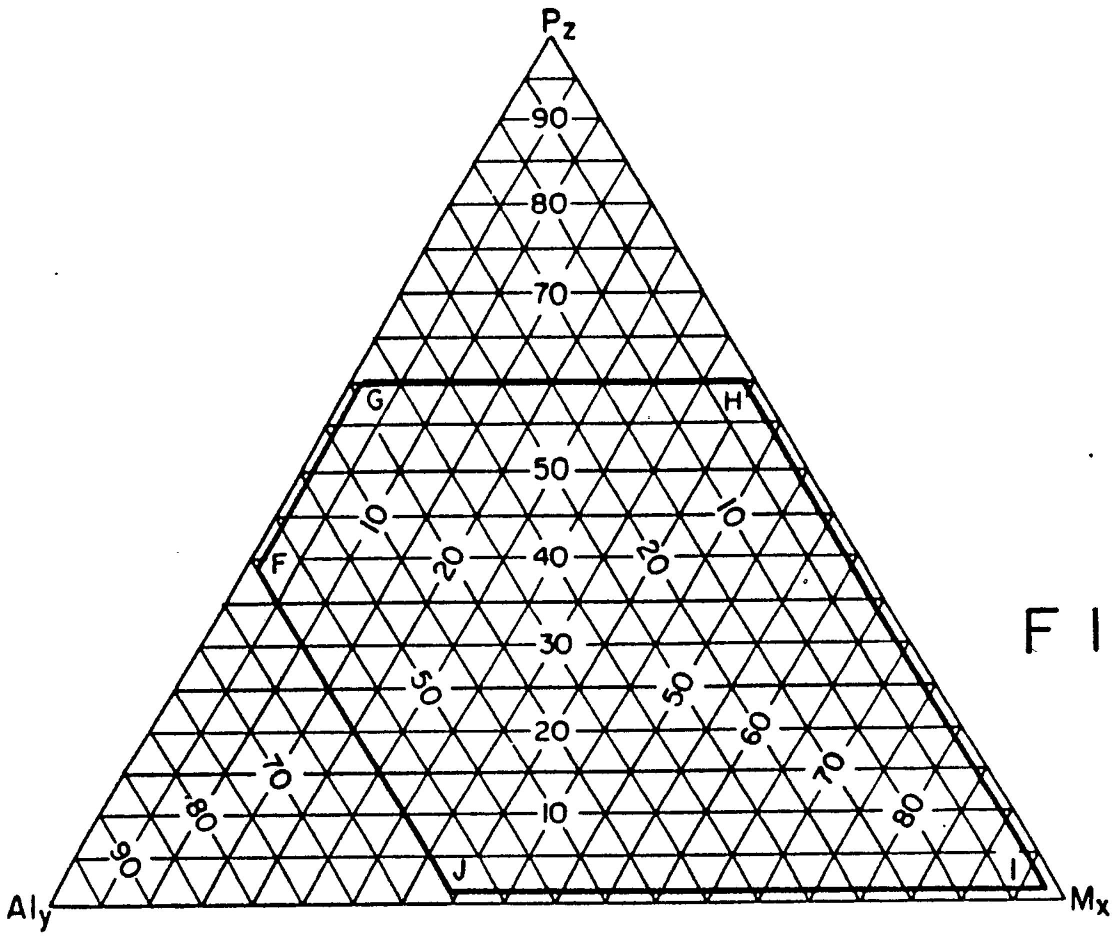
Pz
90
80
70
50
40
30
20
10
10
20
50
70
80
90
10
20
50
60
70
80
G
H
F
J
I
Aly
Mx

FIG. 3